(19) Europäisches Patentamt
European Patent Office
Office européen des brevets

(11) EP 1 051 618 B1

(12) EUROPEAN PATENT SPECIFICATION

(45) Date of publication and mention of the grant of the patent:
**16.09.2009 Bulletin 2009/38**

(21) Application number: **99902181.9**

(22) Date of filing: **12.01.1999**

(51) Int Cl.:
***G01N 33/49*** *(2006.01)*

(86) International application number:
**PCT/US1999/000613**

(87) International publication number:
**WO 1999/035492 (15.07.1999 Gazette 1999/28)**

# (54) METHOD AND APPARATUS FOR DETERMINING BLOOD OXYGEN TRANSPORT

VERFAHREN UND VORRICHTUNG ZUR FESTSTELLUNG DES BLUTSAUERSTOFFTRANSPORTES

PROCEDE ET APPAREIL POUR DETERMINER LE TRANSPORT D'OXYGENE DANS LE SANG

(84) Designated Contracting States:
**AT DE FR GB IT**

(30) Priority: **12.01.1998 US 5474**

(43) Date of publication of application:
**15.11.2000 Bulletin 2000/46**

(73) Proprietors:
- **Buchwald, Henry**
  **Edina, MN 55439 (US)**
- **Menchaca, Hector J.**
  **Minneapolis, MN 55401 (US)**
- **Michalek, Van**
  **Roseville, MN 55113 (US)**
- **O'Dea, Thomas J.**
  **Shoreview, MN 55120 (US)**
- **Rohde, Thomas D.**
  **Minneapolis, MN 55414 (US)**

(72) Inventors:
- **Buchwald, Henry**
  **Edina, MN 55439 (US)**
- **Menchaca, Hector J.**
  **Minneapolis, MN 55401 (US)**
- **Michalek, Van**
  **Roseville, MN 55113 (US)**
- **O'Dea, Thomas J.**
  **Shoreview, MN 55120 (US)**
- **Rohde, Thomas D.**
  **Minneapolis, MN 55414 (US)**

(74) Representative: **Plougmann & Vingtoft A/S**
**Sundkrogsgade 9**
**P.O. Box 831**
**2100 Copenhagen Ø (DK)**

(56) References cited:

**WO-A1-99/21002**
**US-A- 4 013 417**
**US-A- 4 133 874**
**US-A- 4 209 300**
**US-A- 5 686 300**

- **DATABASE WPI Week 9322 Derwent Publications Ltd., London, GB; AN 93-180665 [22] XP002104223 & SU 1 739 295 A (LENGD DOCTORS TRAINING INST), 7 June 1992**
- **DATABASE WPI Week 9739 Derwent Publications Ltd., London, GB; AN 97-423255 XP002104224 & RU 2 073 485 C (PERM MED INST) , 20 February 1997**
- **STEINBACH J.H.; BLACKSHEAR P.L. JR; VARCO R.L.; BUCHWALD H.: 'High blood cholesterol reduces in vitro blood oxygen delivery' THE JOURNAL OF SURGICAL RESEARCH vol. 16, no. 2, February 1974, UNITED STATES, pages 134 - 139, XP007902168**
- **KOYAMA T.; ARAISO T.: 'Diffusion pathway of oxygen in ox lung' ADVANCES IN EXPERIMENTAL MEDICINE AND BIOLOGY vol. 222, 1988, UNITED STATES, pages 63 - 68, XP009082059**
- **KON K.; MAEDA N.; SEKIYA M.; SHIGA T.; SUDA T.: 'A method for studying oxygen diffusion barrier in erythrocytes: effects of haemoglobin content and membrane cholesterol' THE JOURNAL OF PHYSIOLOGY vol. 309, December 1980, ENGLAND, pages 569 - 590, XP007902167**
- **MENCHACA H J. ET AL.: "Decreased blood oxygen diffusion in hypercholesterolemia" SURGERY, vol. 124, no. 4, October 1998 (1998-10), pages 692-698, XP007903155 United States**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 051 618 B1

## Description

### Background of the Invention

The relationship between elevated blood lipids, particularly cholesterol (and especially low-density-lipoprotein cholesterol) and atherosclerosis has been known for many years. More recently, reduction of LDL cholesterol by means of surgery or drugs has been shown to reduce the risk of coronary heart disease. However, the reduction of cardiac events achieved by cholesterol lowering does not correlate well with the relatively small amount of physical regression in the amount of atherosclerotic plaque seen in the coronary arteries following treatment. In addition, relief of angina pectoris (ischemic chest pain) often occurs in a matter of weeks following cholesterol lowering; whereas, documentable changes in the inside diameters of coronary arteries may take years to occur, if they occur at all. The pain associated with angina pectoris is attributable primarily to lactic acid produced when heart muscle cell metabolism occurs in the absence of oxygen. Coronary artery narrowing can limit the amount of blood-transported oxygen that reaches the heart muscle tissue, but, the above observation suggests oxygenation of heart muscle tissue can be improved without increasing blood flow through the coronary vessels.

The way in which changes in blood lipids, such as cholesterol, might affect oxygen delivery to heart muscle tissue has remained unclear. There is abundant oxygen in blood. In fact, oxygenated (arterial) blood contains approximately as many molecules of oxygen per 1000 mL as are found in 200 mL of oxygen gas. Almost all (98-99%) of this oxygen is bound to hemoglobin molecules within the red blood cells; the remainder is physically dissolved in plasma and intracellular red blood cell fluid. For oxygen to reach tissues, such as cardiac muscle tissue, oxygen must be released from hemoglobin and then diffuse across the red blood cell membrane into the plasma and from there into tissues. The movement of oxygen across the red blood cell membrane occurs by passive diffusion and is governed by concentration gradients; there is no active membrane transport system for oxygen. Furthermore, the composition of a subject's red blood cell membrane changes with changes in the subject's lipid status. Therefore, the red blood cell membrane, the immediate surroundings of the red blood cell (the boundary layer), or the contours of the red blood cell membrane can be a significant barrier to release of oxygen into tissue such as cardiac muscle tissue.

What is needed is a method to assess the significance of the red blood cell membrane, the immediate surroundings of the red blood cell (the boundary layer), or the contours of the red blood cell membrane as a hindrance to oxygen transfer from blood to tissues, such as cardiac muscle tissue. Such a method would provide a new way to assess heart and circulatory disorders related to oxygen transport, such as angina pectoris; a new way to measure, and to assess the impact of, factors that affect oxygen transport from a red blood cell, such as a patient's blood lipid levels; and a new way to monitor the effectiveness of lipid-lowering therapies or therapies for improving oxygen transport.

DATABASE WPI Week 9322 Thomson Scientific, London, GB; AN 93-180665 [22] & SU 1 739 295 A (LENGD DOCTORS TRAINING INST) 7 June 1992 discloses an apparatus for obtaining an oxygen dissociation curve. The apparatus comprises a pump, a membrane-type oxygenator and an oxygen meter.

WO 99/21002 shows a system for measuring oxygen in blood which comprises a gas exchange system involving a capillary.

In STEINBACH J H; BLACKSHEAR P L JR; VARCO R L; BUCHWALD H: "High blood cholesterol reduces in vitro blood oxygen delivery" THE JOURNAL OF SURGICAL RESEARCH, vol. 16, no. 2, February 1974, pages 134-139, oxygen transport in general is correlated to the cholesterol level in blood.

KON K; MAEDA N; SEKIYA M; SHIGA T; SUDA T: "A method for studying oxygen diffusion barrier in erythrocytes: effects of haemoglobin content and membrane cholesterol" THE JOURNAL OF PHYSIOLOGY, vol. 309, December 1980, pages 569-590, establishes a link between the rate of oxygen diffusion across a membrane of a red blood cell and the cholesterol content of the membrane of such a cell.

MENCHACA H J. ET AL.: "Decreased blood oxygen diffusion in hypercholesterolemia" SURGERY, vol. 124, no. 4, October 1998, pages 692-698, discloses the measurement of the rate of oxygen diffusion across a membrane of a red blood cell in a blood sample by using an apparatus comprising a closed loop diffusion chamber. Red blood cells were submitted to oxygen and to an environment depleted of oxygen.

### Summary of the Invention

The present invention relates to a method for determining the rate at which oxygen crosses the red blood cell membrane. The method provide a way to assess heart and circulatory disorders related to oxygen transport. Advantageously, the method of the invention can be used to assess a patient's susceptibility to angina pectoris, to determine a patient's blood lipid levels, to measure factors that affect oxygen transport from a red blood cell, and a new way to monitor the effectiveness of lipid-lowering therapy or therapy for improving oxygen transport.

The present method of measures a rate or rates of oxygen diffusion across a red blood cell membrane from the patient. Advantageously, red blood cell samples are standardized to generally uniform conditions of gas content by exposing the red blood cell to oxygen and exposing the red blood cell to an environment depleted of oxygen as part of the measurement process. Preferably, the rate at which oxygen moves across the red blood cell membrane or its boundary layer is determined by monitoring either a blood plasma level of oxygen, a level of oxygen bound to hemoglobin, or both.

The present method of determining a patient's blood lipid level, and its impact, includes measuring a rate or rates of oxygen diffusion across a red blood cell

membrane from the patient. The rate indicates the blood lipid level, for example, through correlating a measured rate with a previously determined rate or range of rates for an established level of blood lipid. Advantageously, red blood cell samples are standardized to generally uniform conditions of gas content by exposing the red blood cell to oxygen and exposing the red blood cell to an environment depleted of oxygen as part of the measurement process. Preferably, the rate at which oxygen moves across the red blood cell membrane is determined by monitoring either a blood plasma level of oxygen, a level of oxygen bound to hemoglobin, or both.

In one embodiment, the method of the invention can be used to assess a patient's susceptibility to angina pectoris. This embodiment includes measuring a rate of oxygen diffusion across a membrane of a red blood cell from the patient.

This rate indicates the patient's susceptibility to angina pectoris, for example, by correlating the measured rate with the susceptibility to angina observed in a control or standardized population, or in the patient, at the measured rate.

In another embodiment, the method of the invention can be used to follow the course of a lipid-lowering therapy. This embodiment includes measuring a rate of oxygen diffusion across a membrane of a red blood cell from the patient. This rate determines the effectiveness of a lipid-lowering therapy, for example, by correlating the measured rate with lipid levels to determine the patient's relative or absolute lipid level, and comparing the patient's lipid level to the patient's previous lipid levels.

An apparatus, which is suitable for conducting the methods of the invention, measures diffusion of oxygen across a red blood cell membrane and includes an oxygen level detector, a gas exchange system, and a red blood cell transport system. The red blood cell transport system is adapted and configured for transporting red blood cells through the gas exchange system and the oxygen level detector. The gas exchange system is adapted and configured to exchange gasses with the red blood cell. The oxygen level detector is adapted and configured for detecting oxygen levels in a red blood cell or in fluid (e.g., plasma) surrounding a red blood cell.

In the apparatus, the oxygen level detector way be a spectrophotometric detector, the red blood cell transport system is a pump, and the gas exchange system is a closed loop diffusion system. The preferred closed loop diffusion system includes gas permeable tubing in a chamber defined by a housing.

The gas permeable tubing has a lumen effective for containing red blood cells and for diffusion of gas through the tubing and to and the red blood cells. The housing is adapted and configured for containing successive samples of gases to effect gas exchange with the red blood cells.

## Brief Description of the Drawings

Figure 1 illustrates three compartments associated with the circulation of blood, involved in oxygen transfer and utilization, and that can be modeled in an apparatus for measuring oxygen levels.

Figure 2 illustrates an embodiment of the apparatus useful in the method of the invention.

Figure 3 is a schematic illustration of a preferred embodiment of an apparatus useful in the method of the invention.

Figure 4 illustrates the plasma oxygen levels for cholesterol-fed and control animals as determined by an embodiment of the method of the invention.

Figure 5 illustrates the correlation of plasma cholesterol and red blood cell membrane cholesterol levels with percent changes per unit time in $O_2$ saturation.

Figure 6 illustrates that groups of patients with ranges of cholesterol levels can also be grouped by the rate at which oxygen diffuses from their red blood cells

Figure 7 illustrates timecourses for oxygen release from red blood cells for patients grouped by cholesterol level.

## Detailed Description of the Invention

The present invention is related to a method for the measurement of the rate of oxygen diffusion across a red blood cell membrane. The method can be employed to monitor treatment of or to diagnose disorders of blood, heart, and/or circulation, such as angina pectoris. The method can also be employed for determining a patient's blood lipid level.

**Oxygen Diffusion Through Red Blood Cell Membranes**

Measuring Oxygen Levels

Oxygen levels in gasses, in liquids, in blood, such as in blood cells or plasma, and in tissues can be measured in several ways and using a variety of instruments that are known in the art. An oxygen electrode detects free molecular oxygen in a liquid and can be used with biological fluids such as blood, plasma, and the like. Oxygen can also be detected by known spectrophotometric methods, either free or as part of a complex with another molecule.

In red blood cells, nearly all oxygen present is complexed with hemoglobin. Such complexes can be detected by numerous methods known in the art, including spectrophotometric methods, fluorometric methods, potentiometric methods, and the like. For example, for absorption of light in the uv/visible range, the greatest difference in absorbance between hemoglobin and oxygenated hemoglobin occurs at 660 nm. At 805 nm, the isobestic point there is no difference in absorbance between oxygenated hemoglobin and hemoglobin. Typically, scattering of light by blood components is accounted for by determining absorbance at a wavelength where neither hemoglobin nor oxygenated hemoglobin significantly absorb light.

After accounting for scattering, the difference in absorbance at 660 nm yields the concentration of oxygenated hemoglobin. Various instruments exist for convenient and automated measurements of levels of oxygenated hemoglobin.

A small amount of the oxygen present in blood is not complexed with hemoglobin, and can be detected as oxygen in plasma. Plasma oxygen can be detected by numerous methods known in the art, including spectrophotometric methods, fluorometric methods, potentiometric methods, and the like. For example, excitation of a plasma sample at 385 nm results in fluorescence of plasma oxygen which is detectable at 515 nm. Light scattering can be taken into account by a measurement at a wavelength outside the range of fluorescence of absorption of oxygen in plasma. Various instruments exist for convenient and automated measurements of levels of plasma oxygen.

Employing one or more instruments that can determine oxygenated hemoglobin and determine plasma oxygen in a system allows both forms of oxygen to be determined in a single sample. Advantageously, in the method of the invention a single instrument or detector can determine both oxygenated hemoglobin and plasma oxygen. Measurement of one or both of the plasma oxygen level and/or the level of oxygenated hemoglobin determines a rate at which oxygen crosses the red blood cell membrane to move from being oxygenated hemoglobin to being plasma oxygen. Either or both of these levels can be monitored continuously or intermittently. Alternatively, measuring an amount or level after a predetermined time period can also yield a rate of diffusion across the red blood cell membrane.

Oxygen in Blood, Tissues, and Model Systems

Figure 1 shows three compartments associated with circulation of blood, that are involved in oxygen transfer and utilization, and that can be modeled in an apparatus for measuring oxygen levels. Oxygen levels can be measured in any or all of these compartments.

Compartment one represents the interior of a red blood cell. A red blood cell lacks a nucleus, organelles, and any internal membranous structures. The cell membrane is the only membrane of a red blood cell; the red blood cell is basically a membranous sack containing hemoglobin. Oxygen in a red blood cell faces only two barriers to exiting the cell: dissociating from hemoglobin and diffusing across the red blood cell membrane. Dissociation from hemoglobin is fast compared to diffusion across the red blood cell membrane. Therefore, the rate at which oxygen leaves a red blood cell reflects the rate at which oxygen diffuses through or across the red blood cell membrane.

The level of oxygen in compartment one is the level of oxygenated hemoglobin in the red blood cell. Only negligible oxygen in a red blood cell is free of hemoglobin. In a red blood cell, total oxygen content can be measured by any of several known methods, for example, by the amount of hematocrit, or hemoglobin and the level of oxygen saturation ($S_{O2}$) of the hemoglobin. The level of oxygen saturation is defined by the concentration of oxygenated hemoglobin [HbO] divided by the concentration of total hemoglobin [Hb] times 100%; [HbO]/ [Hb] x 100%. This can be measured by a variety of methods and instruments known in the art.

Compartment two represents the blood outside of the red blood cell and can include other blood cells, proteins, plasma, serum components, laboratory additives (e.g. anticoagulants), and the like. Compartment two generally contains only a small amount of the total oxygen in blood. However, any oxygen entering or leaving the blood must cross through this compartment on its way to hemoglobin, the oxygen transport vehicle. Therefore, the level of oxygen in compartment two reflects the flux of oxygen from compartment one to compartment three, and also in the reverse direction. Oxygen levels in compartments one and three will affect the oxygen level in and the rate of change of oxygen level in compartment two.

The level of oxygen in compartment two can be represented by plasma oxygen levels. This can be measured as $P_{O2}$, the partial pressure of oxygen in plasma. This measurement can be conducted by a variety of methods and instruments known in the art. Since partial pressure measurements are affected only by gas molecules free in solution, oxygen that is bound to hemoglobin is not included in instantaneous $P_{O2}$ measurements. Over time, however, the hemoglobin does affect $P_{O2}$ values by acting as an oxygen sink, which removes excess oxygen from the plasma when levels are high and replaces plasma oxygen when levels are low.

Compartment three represents the surroundings of a vessel or tube carrying blood. In an animal, compartment three represents tissue that surrounds a blood vessel. Lung tissue supplies oxygen to the blood via diffusion of oxygen through the blood vessel wall and across the membrane of the red blood cell, leading to the formation of oxygenated hemoglobin. Other tissues are nourished by oxygen that dissociates from hemoglobin, crosses the red blood cell membrane, leaves the blood vessel, and enters the tissue. In an apparatus that measures oxygen levels in blood or blood components, compartment three typically represents the surroundings of a tube, such as a gas permeable silicon or silastic tube, carrying blood. In such an apparatus, compartment three can be a gas or liquid (fluid) filled container from which oxygen can diffuse through the tube and into compartments two and one. In addition, in such an apparatus, oxygen can diffuse from compartments one and two into compartment three.

In the apparatus useful in the method of the present invention, the oxygen concentration in compartment three can be controlled. This allows control of the direction and amount of flow of oxygen into and out of compartments one and two. In the method and apparatus of the invention, measuring the amount of oxygen in either or both of compartments one and two reveals the direction and rate of movement of oxygen. For example, depletion of oxygen in compartment three will deplete oxygen in the plasma, and oxygen will dissociate from oxygenated hemoglobin, diffuse through the membrane of the red blood cell and out of the cell. When the concentration of oxygen in compartment three is higher than the concentration in compartment two, the plasma will become oxygenated, and oxygen will diffuse through the membrane of the red blood cell and into the cell, and form oxygenated hemoglobin.

<u>Mathematical Description of Oxygen Diffusion In Tissue and Apparatus</u>

*Blood to Tissue Oxygen Transport*

Although not limiting to the present invention, transport of oxygen from red blood cells to surrounding tissue can be modeled as follows.

For practical calculations, the $O_2$ content of a single red blood cell is difficult to work with. A more suitable estimate of tissue bed $O_2$ availability can be derived by calculations based on a milliliter of whole blood. Under normal physiologic conditions, each gram of hemoglobin (Hb) can bind 1.34 of $O_2$, and each 100 ml of whole blood contains about 15 g of Hb, so that each 100 ml of whole blood can bind up to 20.1 ml of $O_2$ or 0.201 ml $O_2$/ml (20 volume %). Normal arterial blood at a $pO_2$ (partial pressure of $O_2$ dissolved in the blood plasma) of 95.7 mm Hg releases about 0.045 ml of $O_2$/ml blood as the $pO_2$ drops to that of mixed venous blood where the $pO_2$ is 40 mm Hg. This process of unloading takes the $O_2$ saturation of Hb from 97% to 75%. For any Hb value, the $O_2$ concentration of saturated whole blood is equal to 1.34•g Hb/ml. By determining the red blood cell $O_2$ saturation ($SO_2$) the $O_2$ concentration of unsaturated whole blood is equal to $SO_2$•1.34• Hb/ml (1).

In addition to the $O_2$ contained in the red blood cells, the blood contains $O_2$ dissolved in the plasma, which obeys Henry's law, where $[O_2] = \alpha \cdot pO_2 \cdot (1\text{-Hct})$ ($\alpha = 2.04 \cdot 10^{-5}$ ml $O_2$/mm Hg, Hct=hematocrit). At maximum plasma $pO_2$ of 149 mm Hg (1 atmosphere) and a Hct of 40%, about 0.002 ml of $O_2$ can be carried by 1 ml of plasma - about 1% of the carrying capacity of the red blood cell (2). The plasma $O_2$ content is, therefore, negligible. Rather, the primary influence of the plasma $O_2$ content on tissue oxygenation is in the plasma $pO_2$ regulation of $SO_2$.

As stated, the $SO_2$ of arterial blood delivered to the tissues under ordinary conditions is about 97%. From the $O_2$ dissociation curve of Hb, this $SO_2$ corresponds to a $pO_2$ of 95.7 mm Hg. Thus, $[O_2]_{red\ blood\ cell}$ =0.195 ml $O_2$/ml blood, $[O_2]_{plasma}$=0.002 ml $O_2$/ml blood, and $[O_2]_{blood}$=0.197 ml $O_2$/ml blood. The $SO_2$ of mixed venous blood leaving the tissues under ordinary conditions is about 75%, corresponding to a $pO_2$ of about 40 mm Hg. Thus, $[O_2]_{red\ blood\ cell}$ =0.151 ml $O_2$/ml blood, $[O_2]_{plasma}$=0.001 ml $O_2$/ml blood, and $[O_2]_{blood}$=0.152 ml $O_2$/ml blood.

Under conditions of maximum stress and increased tissue $O_2$ demand, the $SO_2$ of arterial blood does not change appreciably. However, the $SO_2$ of mixed venous blood, equal to that of the perfused tissues, does change to a remarkable degree. The $SO_2$ of mixed venous blood leaving the tissues under conditions of maximum stress can go as low as 20% (a $pO_2$ of about 15 mm Hg). Thus, under maximum stress conditions $[O_2]_{red\ blood\ cell}$ =0.040 ml $O_2$/ml blood, $[O_2]_{plasma}$=0.000 ml $O_2$/ml blood, and $[O_2]_{blood}$=0.140 ml $O_2$/ml blood. It follows that the normal $O_2$ delivery of 0.045 ml $O_2$/ml blood rises under maximum stress to 0.157 ml/$O_2$/ml blood, a 3.5-fold increase in tissue $O_2$ availability.

During normal conditions, the average blood flow in the coronary arteries is 1.06 ml/sec or 63.6 ml/min. This flow rate results in an average volume of $O_2$ delivered of 1.06 ml blood/sec•0.045 ml $O_2$/ml blood = 0.048 ml $O_2$sec or 2.86 ml $O_2$/min. During conditions of maximum stress and increased cardiac output, the average blood flow per sec in the coronary arteries can increase 2.39-fold, so that the average blood flow is 2.39•1.06 ml/sec = 2.53 ml/sec or 151.8 ml/min. This flow rate results in an average volume of $O_2$ delivered of 2.53 ml blood/sec•0.157 $O_2$/ml blood = 0.379 ml $O_2$sec or 23.83 ml $O_2$/min. The maximum stress-induced 2.39-fold increase in coronary blood flow is designated the coronary flow reserve factor, which represents 152 ml blood/min in an unimpeded coronary arterial system.

During normal conditions, the area of the heart served by the coronary artery system (the ventricles) needs 1.3 ml $O_2$/min/100 g. During conditions of maximum stress, this value increases to 8.0 ml $O_2$/min/100 g. Reciprocal blood transit times through the heart have been calculated to be 0.2 $sec^{-1}$ to 1.0 $sec^{-1}$ (median, 0.6 $sec^{-1}$). These reciprocal blood transit times correspond to cardiac transit times of 1 to 5 sec (median, 1.6 sec). Under conditions of maximum stress, cardiac transit time decreases to less than 1 sec. The minimum $O_2$ unloading time of normal red blood cell with a membrane thickness of 1 μm has been given as 0.063 sec (2), with more likely times *in vitro* of 0.4 to 1.0 sec (1,5,6). These values are close to the average cardiac transit time, but still lower than cardiac transit time under maximum stress.

*Oxygen Transport in a Gas Exchange Apparatus*

Although not limiting to the present invention, transport of oxygen from a red blood cell to surroundings in a gas exchange apparatus can be modeled as follows.

The requirements described above for delivery of blood through the coronary arteries can be met by a coronary embodiment of the device fitting parameters described below. For example, a coronary embodiment typically will have a $SO_2$ into the apparatus of 97%, corresponding to a $pO_2$ of 95.7 mm Hg; an $SO_2$ out of the apparatus of 75%, corresponding to a $pO_2$ of 40 mm Hg (normal conditions); or an $SO_2$ out of the apparatus of 20%, corresponding to a $pO_2$ of 15 mm Hg (maximum stress conditions). These parameters describe a rate of $O_2$ delivery to the tissue of 2.86 ml $O_2$/min under normal conditions and a rate of $O_2$ delivery to the tissue of 23.83 ml $O_2$/min under conditions of maximum stress. This corresponds to a total $O_2$ delivery to the tissue of about 0.24 ml $O_2$ /pass under normal conditions and a total $O_2$ delivery to the tissue of about 0.40 ml $O_2$/pass under conditions of maximum stress.

A coronary embodiment of the apparatus can be envisioned as having two compartments that are separated by a membrane or tubing barrier permeable to $O_2$ but not to a liquid such as blood plasma: In this embodiment: $V_1$= volume of the sample; $V_2$= volume of the "environment"; $C_1$= oxygen concentration in the sample; $C_2$= oxygen concentration in the "environment"; $\Delta C=C_1-C_2$; A = membrane area; P = membrane permeability to $O_2$; $\Delta x$= membrane or tubing thickness; $(pO_2)_1$= partial pressure of $O_2$ in sample; $(pO_2)_2$= partial pressure of $O_2$ in environment; $\Delta pO_2=(pO_2)_1-(pO_2)_2$; $J_s$=current across membrane; $N_1$= amount of $O_2$ in sample compartment; $N_2$= amount of $O_2$ in environmental compartment; $N=N_1+N_2$=total amount of $O_2$ in system = constant.

Each of these variables can be present in a coronary embodiment including two compartments separated by a barrier permeable to oxygen.

The following equations can describe such a coronary embodiment.

Equation 1:

$$J_s = P \bullet \Delta C = P \bullet \alpha \bullet \Delta pO_2$$

Equation 1 describes the relationship between current across membrane, $O_2$ pressure gradient and concentration gradient. $\alpha$ = relationship between $\Delta C$ and $\Delta pO_2 = 3 \bullet 10^{-5}$ ml $O_2$ /(ml blood •mm Hg). Equation 2:

$$N_1(t) = C_1(t) \bullet V_1 :$$
$$N_2(t) = C_2(t) \bullet V_2 :$$
$$N = N_1 + N_2$$

Equation 2 describes the relationship between amount of $O_2$ and volumes and concentrations in each compartment. Equation 3:

$$dN_1(t)/dt = V_1 \bullet dC_1(t)/dt$$
$$dN_2(t)/dt = V_2 \bullet dC_2(t)/dt$$

Equation 3 describes the relationship between rate of change of amount of $O_2$ and volumes and concentrations in each compartment.

Combining these equations yields equation 4 (remembering that N is a constant):

$$d(\Delta C)/dt = -A \bullet P \bullet [\frac{1}{V_1} + \frac{1}{V_2}] \bullet (\Delta C)$$

Equation 4 is the differential equation derived by adding the equations for continuity and diffusion. Equation 4 has a solution of the form of equation 5:

$$\Delta C(t)/\Delta C(0) = e^{-A \bullet P \bullet [1/V_1 + 1/V_2] \bullet t} = e^{-\frac{t}{t_0}}$$

Equation 5 represents a solution of equation 4 for concentration as a function of time.

Equation 5 can be applied specifically to a coronary embodiment of the present apparatus as follows: In applying it to oxygen transfer through the oxygen porous tubing used in the embodiment from blood or plasma to the environment, the environment has a much larger volume than the tubing and the following approximations hold, $V_2 >> V_1$ and thus $1/V_2 \sim 0$.

The permeability constant of typical gas permeable tubing is shown in equation 6:

$$\Phi = 7961 \bullet 10^{-10} \frac{cm^3 \bullet mm}{cm^2 \bullet sec \bullet cm\,Hg}:$$

Equation 6 describes the value of the permeability constant under physiological conditions for silicone gas permeable tubing material suitable for an apparatus of the invention. Therefore, the time constant is described by equation 7:

$$\frac{1}{t_0} = \frac{A \bullet \Phi}{\Delta x \bullet \alpha \bullet V_1 \bullet 10}$$
$\Delta C$ expressed as $\Delta pO_2$

Equation 7 describes the value a of research apparatus time constant expressed in terms of $pO_2$. One apparatus suitable

for a coronary embodiment can have $\Delta x$ = 0.2415 mm., A= 92.55 $cm^2$, $V_1$=15 ml. And $\alpha$=3.0 $\bullet 10^{-5}$ ml $O_2$/ml blood/mm Hg. Therefore, for such an apparatus, equation 7 yields equation 8:

$$\frac{1}{t_0} = \frac{92.55 \bullet 7916 \bullet 10^{-10}}{0.2415 \bullet 3 \bullet 10^{-5} \bullet 15 \bullet 10}$$
$$\frac{1}{t_0} = 6.78 \bullet 10^{-3}\ sec^{-1}$$

Equation 8 shows the value of a time constant for one apparatus suitable for a coronary embodiment as determined by substitution of suitable values in equation 7. Under typical physiological conditions, equation 7 reduces to equation 9:

$$\frac{\ln\left[\frac{40}{95.7}\right]}{-6.78 \bullet 10^{-3}} = t = 13\ sec$$

Equation 9 illustrates the time of oxygen to leave one apparatus suitable for a coronary embodiment for normal conditions. Under conditions of maximum stress these same equations yield, for a final oxygen pressure of 20 mm Hg, t = 23.1 sec.

Due to several approximations made in this derivation, and known variations in certain of the factors included in these equations, it is believed that the interface between plasma and the gas permeable surface of an apparatus useful in the method of the invention can introduce a t of from 2 to 26 seconds under typical conditions.

The above equations can be readily visualized as applying to blood, that is red blood cells and plasma, in an apparatus by envisioning that equation 5 applies to the diffusion from the red blood cell, with compartment one referring to the red blood cell and 2 referring to the plasma. The volume of the plasma is assumed to be much greater than that of the red blood cell, thus $1/V_2 \approx 0$, as in the above derivation. Further, the $O_2$ content of the plasma can be assumed to be nearly 0, since $\alpha$ is so small. Since the method of the invention and the apparatus useful there in can employ oxygen saturation, $SO_2$, for a measurement of C in a red blood cell, the method and apparatus can relate this to the concentration. Equation 9 leads to equation 10, which shows this relationship. Equation 10:

$$d(\Delta C)/dt = -A \bullet D_0 \bullet [\frac{1}{V_1}] \bullet (\Delta C)$$

Equation 10 is a preliminary step on the way to equation 12 showing Differential equation derived by adding the equations for continuity and diffusion for red blood cells. In equation 10, $D_o$ is the oxygen diffusion coefficient for the red blood cell. The concentration of oxygen relates to the $SO_2$ by equation 11:

$$[O_2] = SO_2 \bullet 1.34 \frac{ml\ O_2}{g\ Hb} \bullet \frac{g\ Hb}{ml\ blood} \approx SO_2 \bullet 20.1 \frac{ml\ O_2}{ml\ blood}$$

Equation 11 describes the relationship between oxygen concentration and oxygen saturation in the red blood cell. This leads to a solution of equation 11 in terms of $SO_2$ shown in equation 12.

$$SO_2(t) = SO_2(0) \bullet e^{-\frac{t}{t_0}} : \frac{1}{t_0} = \frac{A \bullet D_o}{V_1 \bullet 20.1}$$

Equation 12 shows a solution of equation 8 in terms of oxygen saturation in a red blood cell. The value of $D_o$ as $9.5 \bullet 10^{-6}$ $cm^3/sec$ is known to be and the value of $V_1/A$ is approximately $1 \bullet 10^{-4}$ cm.. Thus, equation 13 is:

$$\frac{1}{t_0} = \frac{9.5 \bullet 10^{-6}}{1 \bullet 10^{-4} \bullet 20.1} = 4.73 \bullet 10^{-3}\ sec^{-1}$$

Equation 13 describes the value for the time constant of the diffusion of oxygen from the red blood cell using a large, it is believe the largest reasonable value of $D_o$. A smaller, but still reasonable, value of $D_o$ including the "dead space" around the red blood cell is 2.0 to 4.0 $\bullet 10^{-7}$ $cm^3/sec$. This increases the time constant of the red blood cell as shown in equation 14.

$$\frac{1}{t_0} = \frac{4.0 \bullet 10^{-7}}{1 \bullet 10^{-4} \bullet 20.1} = 2 \bullet 10^{-4}\ sec^{-1}$$

Equation 14 describes the value for the time constant of the diffusion of oxygen from the red blood cell using a smaller, but still reasonable, value of $D_o$.

Applying these values for the red blood cell to a normal case ($SO_2(t)$=75%) and a maximum stress case ($SO_2(t)$=20%), both with initial values of 97 %, the times for discharge are in equations 15 and 16 for the higher value of the diffusion constant and equations 17 and 18 for the lower value of the diffusion constant.

Equation 15:

$$ln\left[\frac{.75}{.97}\right] = -4.73 \bullet 10^{-3} \bullet t : t = 54.3 \text{ seconds}$$

Equation 15 describes the time for red blood cell oxygen diffusion under normal conditions with higher $D_o$. Equation 16:

$$ln\left[\frac{.75}{.97}\right] = -2 \bullet 10^{-4} \bullet t : t = 1286 \text{ seconds}$$

Equation 16 describes the time for red blood cell oxygen diffusion under normal conditions with lower $D_o$. Equation 17:

$$ln\left[\frac{.20}{.97}\right] = -4.73 \bullet 10^{-3} \bullet t\text{: } t = 333.8 \text{ seconds}$$

Equation 17 describes the time for red blood cell oxygen diffusion under maximum stress with higher $D_o$. Equation 18:

$$ln\left[\frac{.20}{.97}\right] = -2 \bullet 10^{-4} \bullet t\text{: } t = 7895 \text{ seconds}$$

Equation 18 describes the time for red blood cell oxygen diffusion under maximum stress with lower $D_o$.

These equations illustrate that the time required for oxygen to diffuse from a red blood cell to the plasma are long compared with the times required for oxygen to diffuse through a tubing or membrane employed in the present invention. Therefore diffusion through such tubing or membrane is fast and does not hinder measurement of rates of diffusion of oxygen through the cholesterol containing membrane of the red blood cell.

Measuring Oxygen Diffusion Across a Red Blood Cell Membrane

The lipid content, particularly the cholesterol content, of the red blood cell membrane is believed to be a factor that affects the diffusion of oxygen through the red blood cell membrane. The cholesterol content of the red blood cell membrane in turn reflects blood cholesterol levels. Therefore, the rate at which oxygen crosses the red blood cell membrane provides a measure of blood cholesterol levels and is useful in diagnosis and treatment of coronary artery disease and other heart and circulatory disorders. The present invention uses the measurement of the rate at which oxygen diffuses across the red blood cell membrane, and includes embodiments directed to methods of evaluating lipid-lowering treatments, methods of diagnosing or assessing the risk of heart and circulatory disorders such as angina pectoris, and methods of determining a patient's blood lipid level.

The present method of determining a patient's blood lipid level form a blood sample typically includes the steps of measuring a rate of oxygen diffusion across a membrane of a red blood cell, and, preferably, correlating the measured rate with established levels of blood lipid to determine the patient's blood lipid level.

The step of measuring the rate of oxygen diffusion across a membrane of a red blood cell preferably includes the steps of exposing the red blood cell to oxygen; exposing the red blood cell to an environment depleted of oxygen; and monitoring either a blood or plasma level of oxygen, a level of oxygen bound to hemoglobin, or both. A blood sample obtained from a patient or subject can contain varying amounts of oxygen, and the rate at which oxygen crosses the red blood cell membrane can, in certain conditions, depend on the amount of oxygen present. Exchanging gasses, either by exposing the red blood cells to oxygen or by exposing the red blood cell to an environment depleted of oxygen, standardizes the blood sample to a predetermined level of oxygen and allows significant comparison of numerous blood samples. The red blood cell can be first exposed to oxygen and subsequently exposed to an environment depleted of oxygen. When exposure to oxygen precedes depletion, oxygen is released from red blood cells during and after depletion, and monitoring, typically, monitors this release. Alternatively, the red blood cell can be first exposed to an environment depleted of oxygen and subsequently exposed to oxygen. When depletion of oxygen precedes exposure to oxygen, oxygen is taken up by the red blood cells during exposure, and monitoring, typically, monitors this uptake.

In a preferred embodiment, exposing the red blood cell to oxygen includes circulating a blood sample in a closed loop diffusion system 17. Typically the closed loop diffusion system 17 includes a chamber 13 containing an atmosphere including oxygen. The level of oxygen in chamber 13 can be varied and be controlled over a wide range. The red blood cells can be exposed to any concentration suitable for standardizing the oxygen level between no oxygen and 100% oxygen. Preferably, the partial pressure of oxygen in chamber 13 is approximately oxygen's partial pressure in air. That is, the atmosphere in chamber 13 includes oxygen at atmospheric gas pressure, for example, 160 mm Hg $O_2$ with 4 mm Hg $CO_2$. Alternatively, the partial pressure of oxygen in chamber 13 can be approximately oxygen's partial pressure in a capillary. That is, the atmosphere in chamber 13 includes oxygen at a pressure, for example, of about 23 mm Hg $O_2$ with 46 mm Hg $CO_2$. Preferably the blood reaches equilibrium with oxygen or with both oxygen and carbon dioxide. In one embodiment, this step of circulating the blood in the closed loop system lasts for about 0.1 to about 60 minutes.

In a preferred embodiment, exposing the red blood cell to an environment depleted of oxygen includes circulating a blood sample in closed loop diffusion system 17, with closed loop diffusion system 17 including chamber 13 containing an atmosphere depleted of oxygen. For example, a suitable oxygen depleted atmosphere is nitrogen or another inert gas, preferably nitrogen. Typically, a commercial or medical grade of nitrogen gas can be employed. Preferably, this depleting step results in complete or nearly complete removal of oxygen from chamber 13, gas permeable tubing 15, and the fluid containing the red blood cells (e.g., plasma). Although considerable deoxygenation is typically observed in the first about 30 seconds, typically, this circulating step lasts longer, preferably, in one embodiment about 15 minutes.

Monitoring either a blood level of oxygen, a level of oxygen bound to hemoglobin, or both can be accomplished employing a variety of methods or instruments, as described herein. Monitoring can take place continuously or intermittently through the exposing and circulating steps, or only at two or more discrete time points. For example, the method can include the step of determining the level of saturation of hemoglobin with oxygen achieved during the step of exposing the red blood cell to oxygen.

In one embodiment, measuring the rate of oxygen diffusion across a red blood cell membrane includes monitoring the ratio of $S_{O2}$/ $P_{O2}$ and plotting this ratio as a function of time under the following conditions:

a) The blood sample is oxygenated, preferably to its maximum, by subjecting compartment three to 1-100% oxygen. Then, $S_{O2}$/ $P_{O2}$, $S_{O2}$, and/or $P_{O2}$ can be measured.

b) The blood sample is subjected to a 0% oxygen environment (e.g., 100% nitrogen or another inert gas) in compartment three. Then, $S_{O2}$/

$P_{O2}$, $S_{O2}$, and/or $P_{O2}$ can be measured, preferably continually, over time. In these conditions, free oxygen has been depleted, but oxygenated hemoglobin remains a source of oxygen. Release of oxygen from oxygenated hemoglobin, which decreases the level of oxygenated hemoglobin, supplies oxygen to the plasma by diffusion through the red blood cell membrane. To the extent that this diffusion is slowed by the membrane, the plasma levels of oxygen ($P_{O2}$) remain depressed and the oxygen saturation ($S_{O2}$) of the hemoglobin remains high for a longer period. Therefore, the rate at which plasma oxygen levels increase, and the rate at which oxygen saturating levels decrease, provide a measure of the rate of diffusion of oxygen through the red blood cell membrane.

<u>Apparatus for Measuring the Rate of Oxygen Diffusion Across a Red Blood Cell Membrane</u>

Figure 2 illustrates an apparatus for measuring the rate of oxygen diffusion across a red blood cell membrane. The apparatus includes an oxygen level detector, a gas exchange system, and a red blood cell transport system. The red blood cell transport system is adapted and configured for transporting red blood cells through the gas exchange system and the oxygen level detector. The gas exchange system is adapted and configured to exchange gasses with the red blood cell. The oxygen level detector is adapted and configured for detecting oxygen levels in a red blood cell or in fluid surrounding a red blood cell.

Oxygen level detector 3 can be any of several detectors suitable for detecting oxygen levels in plasma or another fluid and/or for detecting oxygenated hemoglobin or another oxygen complex. For example, oxygen detector 3 can include an oxygen electrode, a spectrophotometric detector, a fluorometric detector, or a combination of such electrodes and/or detectors. Preferably, oxygen level detector 3 includes detectors for spectrophotometric determination of both plasma oxygen and oxygenated hemoglobin. In another preferred embodiment, oxygen level detector 3 includes detectors for determination of oxygenated hemoglobin. Preferably in one embodiment, oxygen level detector 3 is a dual or multiple wavelength spectrophotometer. Oxygen level detector 3 can be any of a variety of known or commercially available oxygen level detectors.

Preferably, oxygen level detector 3 includes: a light source capable of producing light of 385 nm, 660 nm, 805 nm and an absorption free wavelength; one or more filters to sequentially submit a blood sample to these wavelengths; a cell to allow blood to flow slowly through this light system; and photopickups to detect the transmission of light through the sample at each wavelength. Preferably, oxygen level detector 3 is coupled to appropriate electronics and microprocessors to derive the amounts of, or changes in amounts of, plasma oxygen and/or oxygenated hemoglobin from the comparative signals.

Gas exchange system 5 typically includes a source of gas (not shown), a gas inlet 7, a gas outlet 9, a housing 11 that defines a chamber 13, and a gas permeable tubing 15. These components are typically assembled as a closed loop diffusion system 17. Gas permeable tubing 15 has a lumen (not shown) that is used to contain, preferably flowing, fluid containing red blood cells. A preferred fluid containing red blood cells is blood that has been treated with an anticoagulant. Gas permeable tubing 15 is constructed to allow diffusion of gasses from chamber 13 into the lumen and into any fluid in the lumen and is preferably made of silicone or silastic material. Gas permeable tubing 15 may be a cartridge-type insert which can be easily removed and discarded, after which a new, sterile gas permeable tubing 15 cartridge can be inserted. A removable, and preferably disposable, tubing cartridge increases the testing productivity of the apparatus by drastically decreasing the amount of time lost to cleansing and sterilizing the gas permeable tubing 15 between tests. Further, disposable tubing cartridges decrease the possibility of cross contamination of blood samples, which may lead to inaccurate readings.

Gas is introduced into chamber 13 through gas inlet 7, and exits through gas outlet 9. Preferably, gas flows through chamber 13 to remove any gas that diffuses from gas permeable tubing 15 and to replace any gas the diffuses into gas permeable tubing 15. Housing 11 can be a stoppered laboratory flask, such as an Erlenmeyer flask. Gas exchange system 5 can be any of several suitable systems for exchanging gas into red blood cells, blood, or another fluid.

Red blood cell transport system 19 typically includes a pump 21, inflow tubing 23, and outflow tubing 25. Red blood cell transport system 19 transports plasma or another fluid containing red blood cells through one or more oxygen level detectors 3, into gas exchange system 5, and from gas exchange system 5 back to pump 21. Preferably, pump 21 is a peristaltic pump. Alternatively, red blood cell transport system 19 can include an aspirator, an apparatus that causes flow based on capillary action, or any of several other suitable apparatus for transporting fluids containing red blood cells. Typically, red blood cell transport system 19 includes components necessary for monitoring and recording flow rates and like characteristics as a function of time.

According to the method of the present invention, the apparatus can measure either the plasma oxygen level ($P_{O2}$) or the oxygen saturation ($S_{O2}$) of the hemoglobin, or both.

The time for measuring the oxygen saturation level is typically faster than the measurement time of the plasma oxygen level. Additionally, the rate of change of the oxygen saturation level is faster than the rate of change of the plasma oxygen level, thus an accurate $S_{O2}$ level can be determined in a shorter time period.

The cycle time to perform a test that is, to determine the oxygen diffusion rate across the red blood cell membrane, can be regulated by the components of the apparatus. In particular, the gas permeable tubing 15 can be sized to provide the desired test cycle time. For example, a smaller diameter tubing provides more surface area per volume of blood sample, thereby decreasing the time for diffusion from the blood sample. A decrease in the thickness of the tubing can also decrease the diffusion time. Various shapes of tubing can also decrease the time by increasing the surface area; tubing cross sections such as flat or rectangular shapes provide more surface area per volume than does a circular cross section. A longer tubing length will also increase the surface area. However, the sample size might have to be increased, which can be disadvantageous because a smaller blood sample volume will provide quicker results.

Once the $S_{O2}$ or $P_{O2}$ rate is determined, the apparatus records and/or displays the value. The apparatus may be configured to provide a final rate, a rate at a determined time period, or an average rate. For some tests it may be desirable for the apparatus to provide a continuous rate display or a graphical representation of the rate over time.

Figure 3 is a schematic illustration of a preferred embodiment of an apparatus useful in the method of the invention. This preferred embodiment is illustrated around five components. The gas exchange system 3, includes a chamber 13, which in this embodiment is the first component, an environmental chamber 27. Red blood cell transport system 19 includes the second and third components, a pump system 29 and a sample receiving and diffusion system 31, respectively. The fourth component, a measuring system 33, includes oxygen level detector 3. The fifth component is a control system 35. Each component is coupled, for example, either mechanically or electrically, to one or more of the other components. Each of the components operates cooperatively with one or more of the other components. Such coupling and cooperation is described below. Each of these components in a preferred embodiment can have a variety of preferred characteristics.

For example, a preferred environmental chamber 27 can cycle under one or more of the following sets of conditions. First, at the start of the measurement the chamber houses an oxygen atmosphere at a concentration and in a configuration capable of increasing the $SO_2$ of an about ten ml blood sample in the sample receiving and diffusion system 31 to above about 97.5 % in no more than about 1 minute. Second, for a measurement under normal conditions, the chamber houses an atmosphere of $pO_2$ (about 40 mm Hg) in a configuration capable of decreasing $SO_2$ of an about ten ml blood sample in the sample receiving and diffusion system 31 to about 75% or less in a short time. Third, for a measurement under maximum stress conditions, the chamber houses an atmosphere of $pO_2$ (about 20 mm Hg) in a configuration capable of decreasing $SO_2$ of a ten ml blood sample in the sample receiving and diffusion system 31 to about 40% or less in a short time.

In this context a short time is less than about 20 min, preferably less than about 6 min, preferably less than about 2 min.

A preferred pump system 29 can maintain a steady or rapid pulsatile flow of 10 ml of blood without touching the sample (e.g. through tubing). A preferred pump system 29 includes a control system 35 that can interface with automated (e.g. PC based) laboratory data acquisition and control systems, such as a PC Labview system, or other automated systems as required to control, calibrate, or validate pump system 29. Pump system 29 can pump blood or another fluid containing red blood cells through the sample receiving and diffusion system 31 at a rate sufficient for environmental chamber 27 to effect the diffusion conditions described above.

A preferred sample receiving and diffusion system 31 can receive a sample from an operator of the apparatus and, with power provided by the pump, cycle the sample through environmental chamber 27. A preferred sample receiving and diffusion system 31 can receive an approximately 10 ml or smaller sample of whole blood from a syringe or vacutainer.

Preferably, the blood sample has a volume less than about 3 ml, preferably less than about 1 ml. The pump and receiving and diffusion system 31 work to cycle the blood sample through environmental chamber 27 at a rate sufficient for diffusion as described above. Further, a preferred receiving and diffusion system 31 can be flushed of blood and filled with air or liquid, typically in a short time such as less than one minute.

A preferred receiving and diffusion system 31 is adapted and configured to cooperate with environmental chamber 27 to achieve the oxygen diffusion conditions described above. The time for equilibration under a given sequence of conditions of oxygen diffusion is short enough to provide a convenient test in a medical laboratory. For example, a simple comparative test can be conducted in less than about 20 min, preferably less than about 6 min, more preferably less than about 2 min. A more complex time course test can be conducted and analyzed in less than about 40 min, preferably less than about 15 min, preferably less than about 5 min.

A preferred receiving and diffusion system 31 is adapted and configured as a modular system that reversibly couples to the remainder of the apparatus of the invention. Advantageously, the modular system is constructed and priced to be disposable. For example, such a modular system can reversibly snap or clip into the remainder of the apparatus, fitting similarly to a audio or video cassette into a player. That is, such a modular system can couple both to pump system 29 for pumping fluid through receiving and diffusion system 31, and to measuring system 33 for measuring characteristics (such as oxygen content) of the blood in receiving and diffusion system 31.

For safety in handling blood, a preferred receiving and diffusion system 31 is adapted and configured so that when filling, coupling to the apparatus, or uncoupling from the apparatus, no blood leaves the system. In this way, an operator cannot come into contact with blood from the system.

A preferred measuring system 33 can monitor either $pO_2$ and/or $SO_2$, preferably noninvasively. Advantageously, a measuring system 33 can monitor the $pO_2$ of the gas mixture over a range sufficient to provide the diffusion conditions described above. For example, the range of $pO_2$ is advantageously from about 100 mm Hg to about 20 mm Hg. Advantageously, a measuring system 33 can monitor the $SO_2$ of a blood, or other, sample over a range sufficient to provide the diffusion conditions described above. For example, the range of $SO_2$ is advantageously from about 100% to about 40%, or less. Advantageously, each measurement is made with accuracy and reproducibility sufficient to detect alterations in cholesterol levels produced by therapy such as diet or medicine or to detect alterations in the ability of red blood cells to deliver oxygen to the heart.

Measuring system 33 can measure $SO_2$ or $pO_2$ with a frequency suitable for making simple two point comparisons of values, or for measuring a timecourse of oxygen release or uptake. Measuring system 33 can take a measurement in response to the operator, according to a predetermined program for measurement, by a combination of such procedures, and the like. Advantageously, a measuring system 33 can measure $SO_2$ at least once each 15 seconds in a predetermined program. Preferably, measuring system 33 is adapted and configured for providing a signal communicating the measurement and any associated information to a processor or computer for control and data gathering.

Measuring system 33 can advantageously be calibrated to assure accuracy and precision of measurements of oxygen amounts. For example, a standard solution can be place in position for measurement. The standard solution can be in a specialized calibration module, such as a receiving and diffusion system 31 adapted to contain a standard solution and, advantageously, to communicate to the apparatus that a calibration standard is in the apparatus. Alternatively, a one or more standard solutions can be sequentially added to a typical receiving and diffusion system 31 and the system can be calibrated according to the solution in the system.

The apparatus can be controlled by employing a control system 35 that, for example, controls calibration, display, mechanical actions (e.g. pumping), and measurement by the apparatus. Control system 35 can be manipulated by the operator and/or by a predetermined program to, for example, calibrate the apparatus, monitor that the apparatus is within calibration, start and stop pump system 29 and any other mechanical or electrical systems of the apparatus, recognize a properly inserted receiving and diffusion system 31, and control and communicate with the measurement system.

Control system 35 can incorporate a processor 37 for displaying and performing analysis of measurements taken by the apparatus. For example, advantageously control system 35 can gather $SO_2$ measurement data at least about each 15 seconds and then plot natural log $SO_2$ against time. From such data, a preferred control system 35 can calculate the slope of the plot permeability of red blood cells. Advantageously control system 35 includes data retention apparatus 39 that provides for statistical analysis of any measurements and data, entry of additional patient or clinical information either by the operator or another processor, and the like. Such information can include a hematocrit, cholesterol level, and the like. A preferred control system 35 can display the information and test or measurement data either as a table or graph, and provide output suitable for screen display or printing. Control system 35 can include a screen and/or printer suitable for display and/or printing.

Advantageously control system 35, preferably employing data retention apparatus 39, can store, by methods standard in the data processing arts, patient data either to internal memory or to remote memory, patient data and then correlate patient data from a test with patient data from other, typically previous, tests on the same patient. Alternatively, control system 35 can access a database of population data for patient populations similar to or contrasting with the current patient, and conduct comparison of the current patient data with the population data.

**Oxygen Diffusion, Cholesterol Levels, and Angina**

The rate (or amount in a unit of time) of oxygen diffusion through a red blood cell membrane has been shown to correlate with blood lipid, particularly cholesterol, levels in the cell membrane and in plasma. This knowledge makes the rate of oxygen diffusion though red blood cell membranes useful in treatment and diagnostic regimes for numerous heart or circulatory disorders. Since the present method and apparatus require only a blood sample, they offer an alternative to existing methods, such as arteriography, and are noninvasive and less expensive. In addition, the present device and method allow earlier monitoring of therapy rather than waiting for a noticeable effect on a parameter such as the diameter of a coronary artery.

Treatment of most heart and circulatory disorders involves therapy, such as administration of medicines, directed at lowering a patient's blood lipid levels. Current methods of following the cardiovascular progress of lipid-lowering therapies are expensive and time-consuming. In one embodiment, the method of the invention can be used to follow the course of such lipid-lowering therapy. This embodiment includes measuring a rate of oxygen diffusion across a membrane of a red blood cell of the patient. This rate determines the effectiveness of a lipid-lowering therapy, for example, by correlating the measured rate with lipid levels to determine the patient's relative or absolute lipid level, and comparing the patient's lipid level to the patient's previous lipid levels.

Certain heart and circulatory disorders, such as angina pectoris, have a frequency and severity that correlate with blood levels of cholesterol and like lipids. In one embodiment, the method of the invention can be used to assess a patient's susceptibility to angina pectoris. This embodiment includes measuring a rate of oxygen diffusion across a membrane of a red blood cell from the patient. This rate indicates the patient's susceptibility to angina pectoris, for example, by correlating the measured rate with the susceptibility to angina observed in a control population, or in the patient, at the measured rate.

Angina can also be related to insufficient delivery of oxygen to the tissue of the heart. Under high stress blood is in the arteries supplying the heart for a shorter time than during periods of low stress. Therefore, the rate at which oxygen diffuses out of the red blood cell and the blood vessel may be too slow to release oxygen during the short residence time in the heart during high stress. This rate may be sufficient to deliver oxygen to the tissue during the longer residence times of low stress. Thus, the present method can provide another way to assess susceptibility to angina based on the correlation with residence time of blood in the heart. This method also includes measuring a rate of oxygen diffusion across a membrane of a red blood cell from the patient. This rate indicates the patient's susceptibility to angina pectoris, for example, by correlating the measured rate with the susceptibility to angina observed in a control population, or in the patient, at the measured rate, and, optionally, correlating the measured rate with residence time of blood in the heart during stress.

The present invention may be better understood with reference to the following examples. These examples are intended to be representative of specific embodiments of the invention, and are not intended as limiting the scope of the invention.

**Examples**

**Example 1 -- Correlation of Cholesterol Levels With**

**Red Blood Cell Oxygen Diffusion in an Animal Model**

This study determined a correlation between the level of a blood lipid, cholesterol, and the rate at which oxygen diffused out of red blood cells.

Materials and Methods

Ten New Zealand White Rabbits were divided into an experimental group and a control group. The six experimental rabbits were fed for eight weeks a diet of standard laboratory rabbit chow supplemented with 0.25% cholesterol. The four control rabbits received, for the same period, the same diet lacking the added cholesterol. After eight weeks on this diet, blood samples were collected from each rabbit by standard methods using sodium heparin as an anticoagulant. The plasma and red blood cell cholesterol levels were determined in an aliquot of each blood sample by the Allain's assay and Abell's methods, each of whichs is a standard method.

Another aliquot of each blood sample was circulated through a closed loop diffusion chamber in gas permeable tubing and exposed to atmospheric pressures of oxygen (160 mm Hg) and carbon dioxide (4 mm Hg) for 6 minutes (time 6-12 minutes in Figure 4). This was considered full saturation of the blood with oxygen. Each blood sample was then subjected to desaturation by circulating the blood sample through the closed loop diffusion chamber and exposing the sample to nitrogen gas for 15 minutes (time 12-27 minutes in Figure 4). During exposure to oxygen and during exposure to nitrogen, each sample was subjected to continuous blood gas monitoring for pH, $P_{CO2}$, and $P_{O2}$.

Results

The results of this study are shown in Table 1 and Figure 4. Table 1 illustrates that the experimental, cholesterol-fed animals had higher levels of cholesterol both in their plasma and in their red blood cell membranes than the control animals.

This higher level of cholesterol in plasma and in red blood cell membranes correlated with slower diffusion of oxygen through the red blood cell membrane (Figure 4). Figure 4 shows that the cholesterol-fed animals achieved higher levels of plasma oxygen during the saturation phase due to slower uptake by the red blood cells. When the cells were exposed to the nitrogen atmosphere, oxygen was exchanged out of the cholesterol-fed rabbit plasma more quickly than the control rabbit plasma. This indicates that red blood cell oxygen diffused more slowly into the plasma from the red blood cells from the cholesterol-fed rabbits than in the control blood.

Table 1 -- Cholesterol levels in rabbit plasma and red blood cell membranes in control and experimental groups after eight weeks of feeding.

| | Cholesterol (mg/dl) | |
|---|---|---|
| Group | Plasma | Red Blood Cell Membrane |
| | Mean SEM | Mean SEM |
| Control | 60 ± 1.2 | 22 ± 1.7 |
| Cholesterol | 928 ± 31* | 121 ± 3* |
| * p<0.05 vs. Control Group | | |

Conclusion

Oxygen diffused more slowly across the red blood cell membranes of animals with the higher level of cholesterol in plasma or in red blood cell membrane. This indicates that the rate of diffusion of oxygen across a red blood cell membrane correlates with increased levels of the blood lipid cholesterol in an animal model commonly used in this field for study of blood lipids.

**Example 2 -- Correlation of Cholesterol Levels With Red Blood Cell Oxygen Diffusion in Humans**

**Study 1**

This study determined a correlation between the level of a blood lipid, cholesterol, and the amount of oxygen that diffused into human red blood cells in 15 minutes.

Materials and Methods

Blood samples were collected by standard methods from four informed human volunteers with varying cholesterol levels. Cholesterol levels were determined in one aliquot of each blood sample by Abell's assay, a standard method. Another four aliquots from each blood sample were subjected to blood gas analysis as follows: Each aliquot was subjected to desaturation as described in Example 1 and the amount of oxygen bound to hemoglobin (Hb) was determined. Then, the aliquot was circulated through a diffusion chamber and exposed to capillary gas pressures, 23 mm Hg of $O_2$ and 46 mm Hg $CO_2$. After 15 minutes of circulation, the amount of oxygen bound to hemoglobin (Hb) was determined again.

Results

The results of this study are presented in Table 2. The results presented in Table 2 show that the amount of oxygen that crossed the red blood cell membrane decreased as the cholesterol level increased.

Table 2 - Correlation with cholesterol levels of amounts of oxygen bound to hemoglobin in human red blood cells before and after exposure to oxygen.

| Sample | P Chol (mg/dl) | $O_2$ Content (ml/gm of Hb) Pre-Diffusion Mean ± SEM | Post-Diffusion Mean ± SEM | % Change | *p* Value |
|---|---|---|---|---|---|
| A | 87 | 13.3 ± 0.391 | 20.5 ± 0.478 | 35% | 0.037 |
| B | 157 | 14.8 ± 0.091 | 19.5 ± 0.270 | 24% | 0.041 |
| C | 241 | 15.8 ± 0.013 | 20.2 ± 0.551 | 22% | 0.020 |
| D | 400 | 16.3 ± 0.079 | 17.5 ± 0.196 | 7% | 0.014 |

Conclusion

Oxygen diffused more quickly across the red blood cell membranes of humans with the lower level of cholesterol. This indicates that the rate of diffusion of oxygen across a red blood cell membrane correlates inversely with increasing levels of the blood lipid cholesterol in humans.

**Study 2**

This study determined that plasma cholesterol levels and red blood cell membrane cholesterol levels in humans inversely correlate with the rate of oxygen diffusion from the human subject's red blood cell.

Materials and Methods

In this second study, venous blood was collected from 22 volunteers, standardized to a hematocrit level of the blood of 40%, and circulated in a closed-loop $O_2$ diffusion chamber to full saturation and subjected it to desaturation, while continuously measuring the $O_2$ saturation.

Results

$O_2$ diffusion from inside to outside the red blood cell was represented by the percent change of $O_2$ saturation in a controlled time interval. The plasma cholesterol and red blood cell membrane cholesterol levels were inversely correlated with the percent changes in $O_2$ saturation: $R^2$=0.2996 and $R^2$=0.3870, respectively (Figure 5).

Conclusions

Again, plasma cholesterol and red blood cell membrane cholesterol levels inversely correlated with the trans-red blood cell -membrane $O_2$ diffusion rate, and high blood cholesterol restricted $O_2$ transport.

**Study 3**

This study determined that groups of patients with ranges of cholesterol levels can also be grouped by the rate at which oxygen diffuses from their red blood cells.

Materials and Methods

In this third study, red blood cell $O_2$ diffusion was studied as described above in blood from 54 volunteers, whose blood hematocrit was again standardized to 40%. Patients were grouped by plasma cholesterol (mg/dl) of <199 (n=11, 200 to 224 (n=15), 225 to 249 (n=23), and 275 to 299 (n=5).

Results

The results of this study are reported in Figure 6. Figure 6 illustrates that the 3 plasma cholesterol groups > 200 mg/dl all had marked $O_2$ diffusion reductions, compared with the <199 mg/dl group, at 1 min: 82% (p=0.080), 70% (p=0.036), and 100% (p=0.012), respectively; and at 2 min: 32% (p=0.05), 45% (p=0.008), and 66% (p=0.001), respectively. When the hypoxic conditions were maintained over 12 min, the $O_2$ diffusion depravation induced by hypercholesterolemia was cumulative.

Conclusions

This study determined that groups of patients with ranges of cholesterol levels can also be grouped by the rate at which oxygen diffuses from their red blood cells.

**Study 4**

This study provided an extended analysis of groups of patients with ranges of cholesterol levels and determined short measurement times that revealed different oxygen diffusion rates from red blood cells.

Materials and Methods

This study was an extended analysis involving 93 patents. The patients were grouped into 5 quintiles by plasma cholesterol concentrations: 175 to 199 mg/dl, 200 to 224 mg/dl, 225 to 249 mg/dl, 250 to 274 mg/dl, and 275 to 299 mg/dl.

Results

The 5 cholesterol groups layered out as expected with respect to the percent change in blood $O_2$ diffusion. The greatest percentage change occurred in the lowest cholesterol group; the least percent change in the highest cholesterol group.

Conclusions

A very clear differentiation between these groups could be seen within the first 2 min of circulation in an apparatus as described above the equivalent of about 2 sec of cardiac circulation (Figure 7).

## Claims

**1.** A method for determining a patient's blood oxygen transport and lipid level from a blood sample, comprising the steps of:

measuring a rate of oxygen diffusion across a membrane of a red blood cell of the blood sample; and
correlating the measured rate with established levels of blood lipid to determine the patient's blood lipid level.

**2.** The method of claim 1, wherein the step of measuring comprises:

exposing the red blood cell to oxygen;
exposing the red blood cell to an environment depleted of oxygen; and
monitoring either a blood level of oxygen, a level of oxygen bound to hemoglobin, or both.

**3.** The method of claim 2, wherein exposing the red blood cell to oxygen comprises circulating a blood sample in a closed loop diffusion chamber, the chamber housing an atmosphere comprising oxygen.

**4.** The method of claim 3, wherein the atmosphere comprising oxygen comprises atmospheric gas pressures.

**5.** The method of claim 4, wherein the gas pressures comprises about 160 mm Hg $O_2$ and about 4 mm Hg $CO_2$.

**6.** The method of claim 3, wherein the atmosphere comprising oxygen comprises capillary gas pressures.

**7.** The method of claim 6, wherein the gas pressures comprise about 23 mm Hg $O_2$ and about 46 mm Hg $CO_2$.

**8.** The method of claim 3, wherein circulating lasts for about 6 min.

**9.** The method of claim 2, wherein exposing the red blood cell to an environment depleted of oxygen comprises circulating a blood sample in a closed loop diffusion chamber, the chamber housing an atmosphere comprising nitrogen and depleted of oxygen.

**10.** The method of claim 9, wherein the atmosphere is supplied from a container of commercial grade nitrogen gas.

**11.** The method of claim 9, wherein circulating lasts for about 15 min.

**12.** The method of claim 2, wherein the step of exposing the red blood cell to oxygen precedes the step of exposing the red blood cell to an environment depleted of oxygen.

**13.** The method of claim 2, wherein the step of exposing the red blood cell to an environment depleted of oxygen precedes the step of exposing the red blood cell to oxygen.

**14.** The method of claim 1, wherein the measuring step is performed on a whole blood sample comprising anticoagulant.

**15.** A method for determining a patient's susceptibility to angina pectoris from a blood sample, comprising the steps of:

measuring a rate of oxygen diffusion across a membrane of a red blood cell of the blood sample; and
correlating the measured rate with the susceptibility to angina pectoris observed at the measured rate. in a control population, or observed previously in the patient.

**16.** The method of claim 15, wherein the step of measuring comprises:

exposing the red blood cell to oxygen;
exposing the red blood cell to an environment depleted of oxygen; and
monitoring either a blood level of oxygen, a level of oxygen bound to hemoglobin, or both.

**17.** A method for determining the effectiveness of a lipid-lowering therapy from a patient's blood sample, comprising the steps of:

measuring a rate of oxygen diffusion across a membrane of a red blood cell of the blood sample;
correlating the measured rate with established levels of blood lipid to determine the patient's relative or absolute blood lipid level; and
comparing the patient's lipid level to the patient's previous lipid level.

**18.** The method of claim 17, wherein the step of measuring comprises:

exposing the red blood cell to oxygen;
exposing the red blood cell to an environment depleted of oxygen; and
monitoring either a blood level of oxygen, a level of oxygen bound to hemoglobin, or both.

**Patentansprüche**

**1.** Verfahren zur Bestimmung des Blutsauerstofftransports und des Lipidniveaus eines Patienten auf der Grundlage einer Blutprobe, welches Verfahren folgende Schritte umfasst:

Messen einer Geschwindigkeit von Sauerstoffdiffusion quer durch eine Membrane einer roten Blutzelle der Blutprobe; und
Korrelierung der gemessenen Geschwindigkeit mit Hilfe von festgesetzten Niveaus von Blutlipid zur Bestimmung des Blutlipidniveaus des Patienten.

**2.** Verfahren nach Anspruch 1, wo der Messschritt umfasst, dass:

die rote Blutzelle Sauerstoff ausgesetzt wird;
die rote Blutzelle einem Milieu ausgesetzt wird, das von Sauerstoff erschöpft ist; und
ein Blutsauerstoffniveau, ein Niveau von Sauerstoff, der an Hämoglobin gebunden ist, oder beides überwacht wird.

3. Verfahren nach Anspruch 2, wo die rote Blutzelle Sauerstoff ausgesetzt wird, und dies umfasst, dass eine Blutprobe in einer geschlossenen Lochdiffusionskammer in Umlauf gebracht wird, welche Kammer eine Sauerstoff umfassende Atmosphäre enthält.

4. Verfahren nach Anspruch 3, wo die Sauerstoff umfassende Atmosphäre atmosphärische Gasdrücke umfasst.

5. Verfahren nach Anspruch 4, wo die Gasdrücke etwa 160 mm Hg $O_2$ und etwa 4 mm Hg $CO_2$ umfassen.

6. Verfahren nach Anspruch 3, wo die Sauerstoff umfassende Atmosphäre Kapillargasdrücke umfasst.

7. Verfahren nach Anspruch 6, wo die Gasdrücke etwa 23 mm Hg $O_2$ und etwa 46 mm Hg $CO_2$ umfassen.

8. Verfahren nach Anspruch 3, wo das Umlaufen etwa 6 Minuten dauert.

9. Verfahren nach Anspruch 2, wo die rote Blutzelle einem Milieu, das von Sauerstoff erschöpft ist, ausgesetzt wird, und dies umfasst, dass die Blutprobe in einer geschlossenen Lochdiffusionskammer in Umlauf gebracht wird, welche Kammer eine Stickstoff umfassende Atmosphäre, die von Sauerstoff erschöpft ist, enthält.

10. Verfahren nach Anspruch 9, wo die Atmosphäre aus einem Behälter, der Stickstoff kommerzieller Qualität enthält, zugeführt wird.

11. Verfahren nach Anspruch 9, wo das Umlaufen etwa 15 Minuten dauert.

12. Verfahren nach Anspruch 2, wo der Schritt, wo die rote Blutzelle Sauerstoff ausgesetzt wird, dem Schritt vorausgeht, wo die rote Blutzelle einem Milieu, das von Sauerstoff erschöpft ist, ausgesetzt wird.

13. Verfahren nach Anspruch 2, wo der Schritt, wo die rote Blutzelle einem Milieu, das von Sauerstoff erschöpft ist, ausgesetzt wird, dem Schritt vorausgeht, wo die rote Blutzelle Sauerstoff ausgesetzt wird.

14. Verfahren nach Anspruch 1, worin der Messschritt an einer Antikoagulationsstoff umfassenden Vollblutprobe ausgeführt wird.

15. Verfahren zur Bestimmung der Empfindlichkeit eines Patienten gegen Angina Pectoris auf der Grundlage einer Blutprobe, welches Verfahren folgende Schritte umfasst:

   Messen einer Geschwindigkeit von Sauerstoffdiffusion quer durch eine Membrane einer roten Blutzelle der Blutprobe; und
   Korrelierung der gemessenen Geschwindigkeit mit der bei einer Kontrollpopulation oder bei dem Patienten observierten Empfindlichkeit gegen Angina Pectoris bei der gemessenen Geschwindigkeit.

16. Verfahren nach Anspruch 15, wo der Messschritt umfasst, dass:

   die rote Blutzelle Sauerstoff ausgesetzt wird;
   die rote Blutzelle einem Milieu ausgesetzt wird, das von Sauerstoff erschöpft ist; und
   ein Blutsauerstoffniveau, ein Niveau von Sauerstoff, der an Hämoglobin gebunden ist, oder beides überwacht wird.

17. Verfahren zur Bestimmung der Wirksamkeit einer lipidsenkenden Therapie auf der Grundlage einer Blutprobe eines Patienten, welches Verfahren folgende Schritte umfasst:

   Messen einer Geschwindigkeit von Sauerstoffdiffusion quer durch eine Membrane einer roten Blutzelle der Blutprobe;
   Korrelierung der gemessenen Geschwindigkeit mit Hilfe von festgesetzten Niveaus von Blutlipid zur Bestimmung des relativen oder absoluten Blutlipidniveaus des Patienten; und
   Vergleich des Lipidniveaus des Patienten mit dem früheren Lipidniveau des Patienten.

18. Verfahren nach Anspruch 17, wo der Messschritt umfasst, dass:

die rote Blutzelle Sauerstoff ausgesetzt wird;
die rote Blutzelle einem Milieu ausgesetzt wird, das von Sauerstoff erschöpft ist; und
ein Blutsauerstoffniveau, ein Niveau von Sauerstoff, der an Hämoglobin gebunden ist, oder beides überwacht wird.

**Revendications**

1. Une méthode de détermination du transport d'oxygène sanguin et du taux de lipides sanguins d'un patient à partir d'un échantillon de sang, la méthode comprenant les étapes suivantes :

   mesure d'une vitesse de diffusion de l'oxygène au travers d'une membrane d'un globule rouge de l'échantillon de sang ; et
   corrélation de la vitesse mesurée avec des taux de lipides sanguins établis afin de déterminer le taux de lipides sanguins du patient.

2. La méthode de la revendication 1, dans laquelle l'étape de mesure comprend :

   exposition du globule rouge à de l'oxygène ;
   exposition du globule rouge à un environnement appauvri en oxygène ; et
   surveillance d'un taux d'oxygène sanguin, ou d'un taux d'oxygène lié à l'hémoglobine, ou des deux.

3. La méthode de la revendication 2, dans laquelle l'exposition du globule rouge à de l'oxygène comprend la circulation d'un échantillon de sang dans une chambre de diffusion en circuit fermé, la chambré renfermant une atmosphère comprenant de l'oxygène.

4. La méthode de la revendication 3, dans laquelle l'atmosphère comprenant de l'oxygène comprend des pressions de gaz atmosphériques.

5. La méthode de la revendication 4, dans laquelle les pressions de gaz comprennent environ 160 mm de Hg $O_2$ et environ 4 mm de Hg $CO_2$.

6. La méthode de la revendication 3, dans laquelle l'atmosphère comprenant de l'oxygène comprend des pressions de gaz capillaires.

7. La méthode de la revendication 6, dans laquelle les pressions de gaz comprennent environ 23 mm de Hg $O_2$ et environ 46 mm de Hg $CO_2$.

8. La méthode de la revendication 3, dans laquelle la circulation dure environ 6 minutes.

9. La méthode de la revendication 2, dans laquelle l'exposition du globule rouge à un environnement appauvri en oxygène comprend la circulation d'un échantillon de sang dans une chambre de diffusion en circuit fermé, la chambre renfermant une atmosphère comprenant de l'azote et appauvrie en oxygène.

10. La méthode de la revendication 9, dans laquelle l'atmosphère provient d'un conteneur d'azote gazeux de qualité commerciale.

11. La méthode de la revendication 9, dans laquelle la circulation dure environ 15 minutes.

12. La méthode de la revendication 2, dans laquelle l'étape d'exposition du globule rouge à de l'oxygène précède l'étape d'exposition du globule rouge à un environnement appauvri en oxygène.

13. La méthode de la revendication 2, dans laquelle l'étape d'exposition du globule rouge à un environnement appauvri en oxygène précède l'étape d'exposition du globule rouge à de l'oxygène.

14. La méthode de la revendication 1, dans laquelle l'étape de mesure est réalisée sur un échantillon de sang total comprenant un anticoagulant.

**15.** Une méthode de détermination de la prédisposition d'un patient à l'angine de poitrine à partir d'un échantillon de sang, comprenant les étapes suivantes :

mesure d'une vitesse de diffusion de l'oxygène au travers d'une membrane d'un globule rouge de l'échantillon de sang ; et
corrélation de la vitesse mesurée avec la prédisposition à l'angine de poitrine observée à la vitesse mesurée chez une population témoin, ou observée antérieurement chez le patient.

**16.** La méthode de la revendication 15, dans laquelle l'étape de mesure comprend :

exposition du globule rouge à de l'oxygène ;
exposition du globule rouge à un environnement appauvri en oxygène ; et
surveillance d'un taux d'oxygène sanguin, ou d'un taux d'oxygène lié à l'hémoglobine, ou des deux.

**17.** Une méthode de détermination de l'efficacité d'un traitement hypolipémiant à partir d'un échantillon de sang d'un patient, comprenant les étapes suivantes :

mesure d'une vitesse de diffusion de l'oxygène au travers d'une membrane d'un globule rouge de l'échantillon de sang ;
corrélation de la vitesse mesurée avec des taux de lipides sanguins établis afin de déterminer le taux de lipides sanguins absolu ou relatif du patient ; et
comparaison du taux de lipides du patient au taux de lipides antérieur du patient.

**18.** La méthode de la revendication 17, dans laquelle l'étape de mesure comprend :

exposition du globule rouge à de l'oxygène ;
exposition du globule rouge à un environnement appauvri en oxygène ; et
surveillance d'un taux d'oxygène sanguin, ou d'un taux d'oxygène lié à l'hémoglobine, ou des deux.

FIG.1

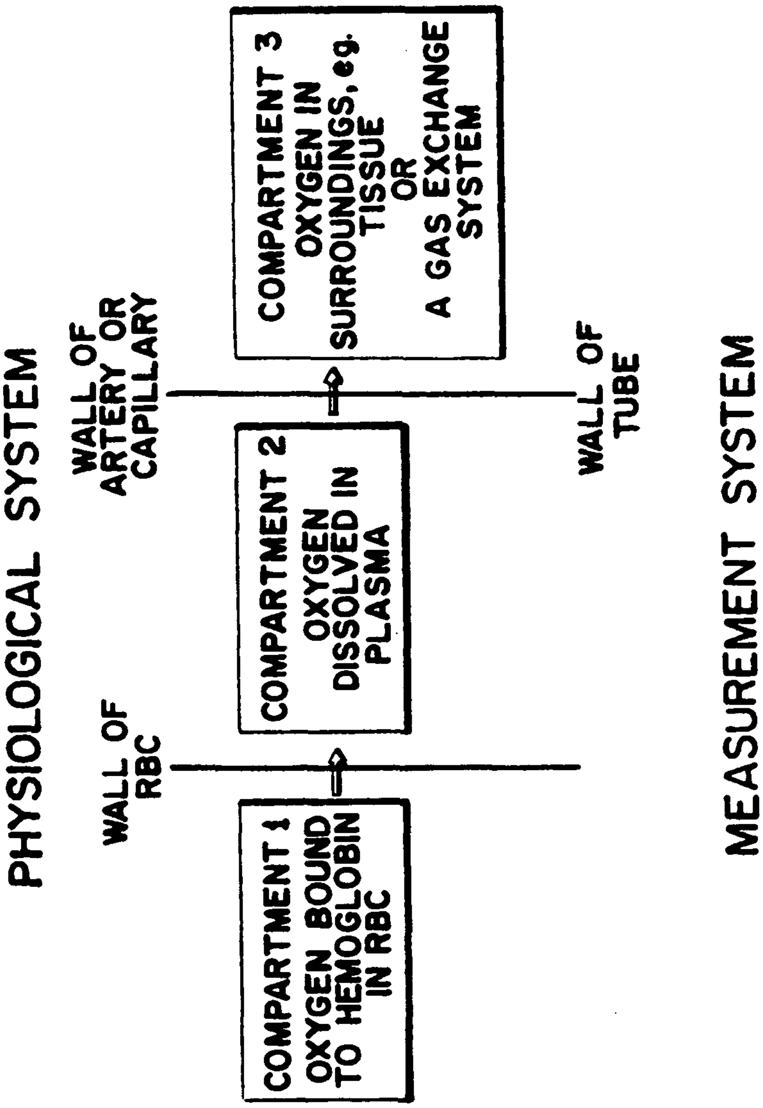
PHYSIOLOGICAL SYSTEM
WALL OF RBC
WALL OF ARTERY OR CAPILLARY
COMPARTMENT 1 OXYGEN BOUND TO HEMOGLOBIN IN RBC
COMPARTMENT 2 OXYGEN DISSOLVED IN PLASMA
COMPARTMENT 3 OXYGEN IN SURROUNDINGS, eg. TISSUE OR A GAS EXCHANGE SYSTEM
WALL OF TUBE
MEASUREMENT SYSTEM

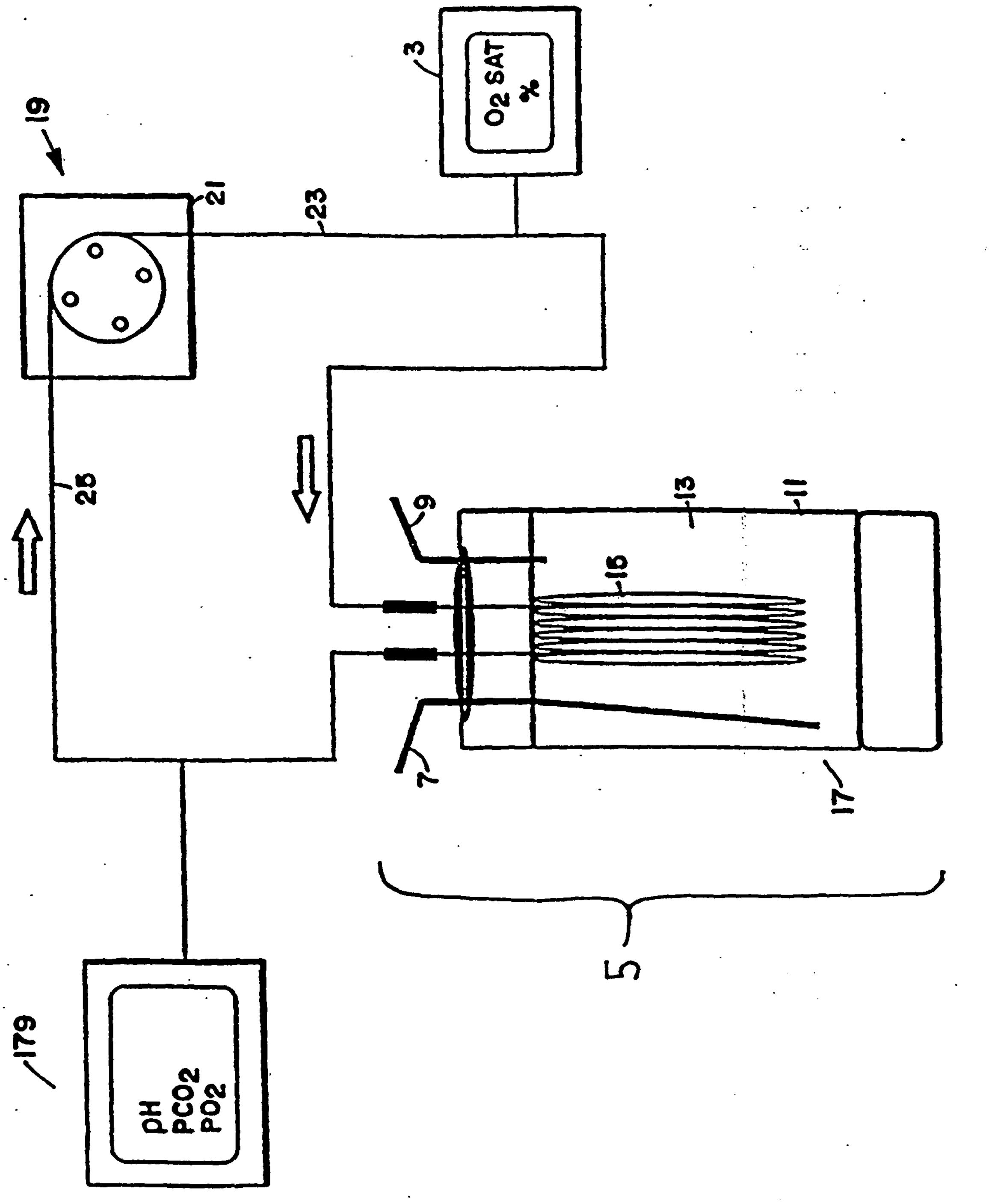


FIG. 2

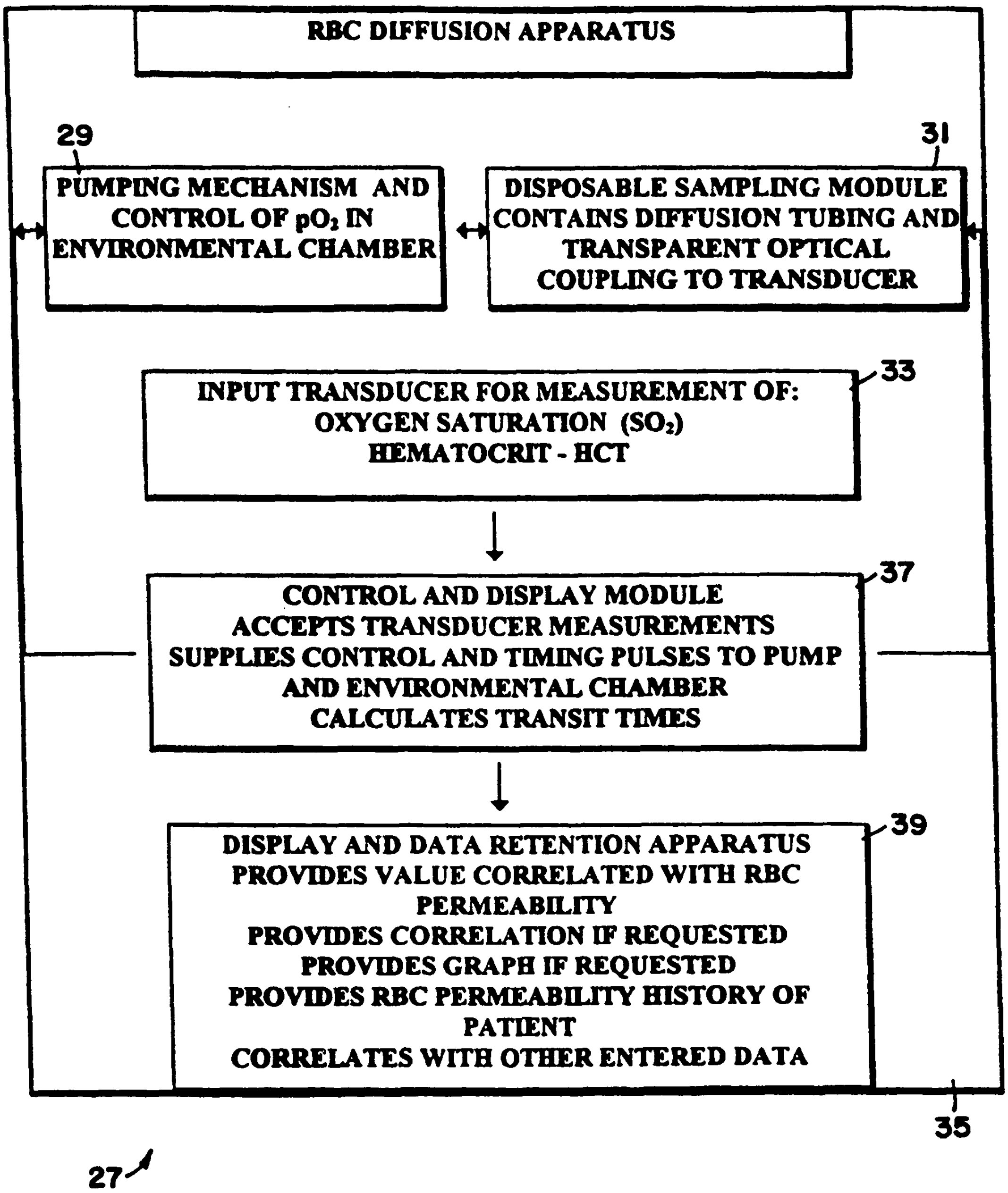
RBC DIFFUSION APPARATUS
29
PUMPING MECHANISM AND CONTROL OF $pO_2$ IN ENVIRONMENTAL CHAMBER
31
DISPOSABLE SAMPLING MODULE CONTAINS DIFFUSION TUBING AND TRANSPARENT OPTICAL COUPLING TO TRANSDUCER
33
INPUT TRANSDUCER FOR MEASUREMENT OF: OXYGEN SATURATION ($SO_2$) HEMATOCRIT - HCT
37
CONTROL AND DISPLAY MODULE ACCEPTS TRANSDUCER MEASUREMENTS SUPPLIES CONTROL AND TIMING PULSES TO PUMP AND ENVIRONMENTAL CHAMBER CALCULATES TRANSIT TIMES
39
DISPLAY AND DATA RETENTION APPARATUS PROVIDES VALUE CORRELATED WITH RBC PERMEABILITY PROVIDES CORRELATION IF REQUESTED PROVIDES GRAPH IF REQUESTED PROVIDES RBC PERMEABILITY HISTORY OF PATIENT CORRELATES WITH OTHER ENTERED DATA
35
27

FIG. 3

FIG. 4

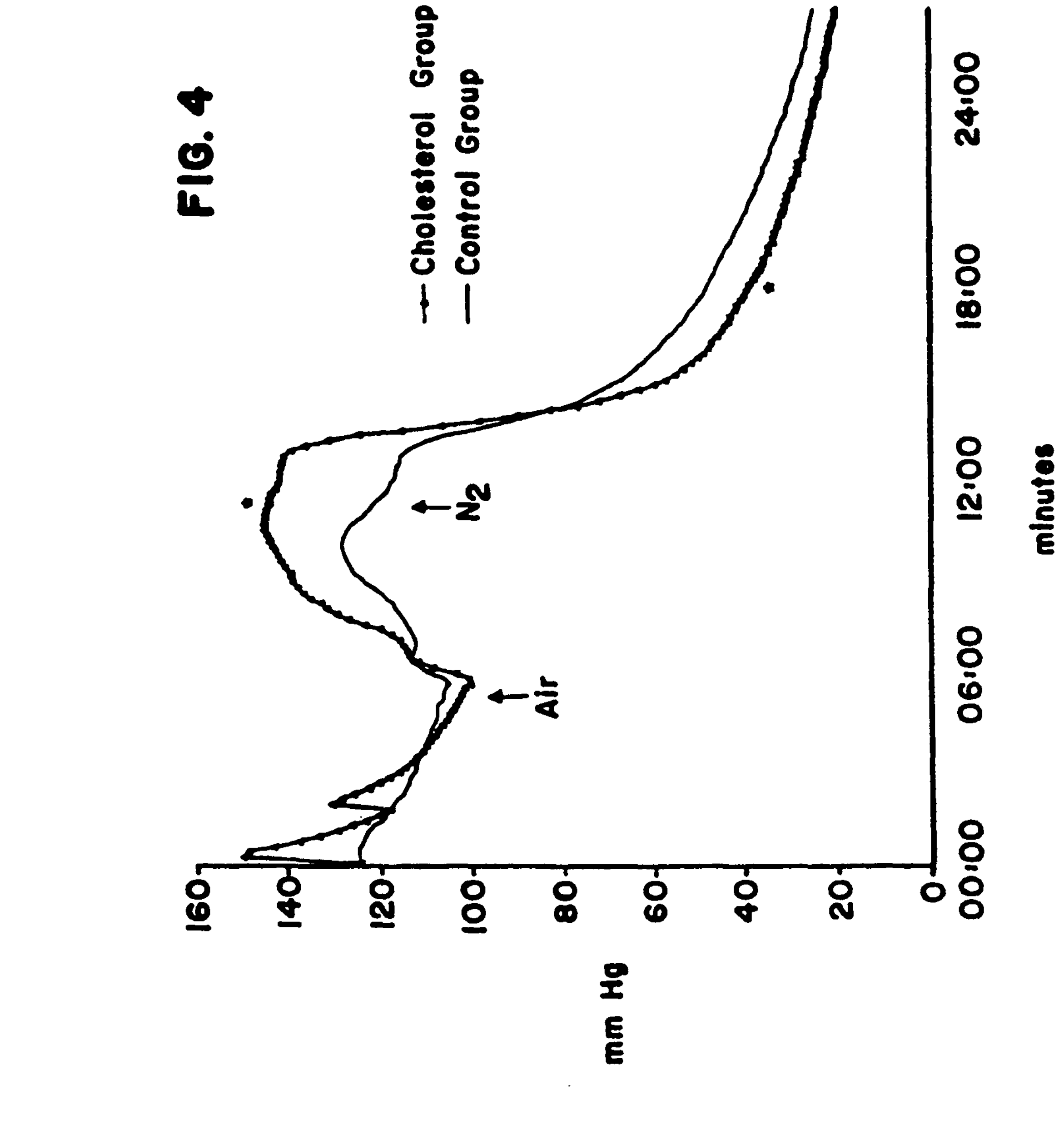
Cholesterol Group
Control Group
mm Hg
160
140
120
100
80
60
40
20
0
00:00
06:00
12:00
18:00
24:00
minutes
Air
N2

**FIG. 5A**

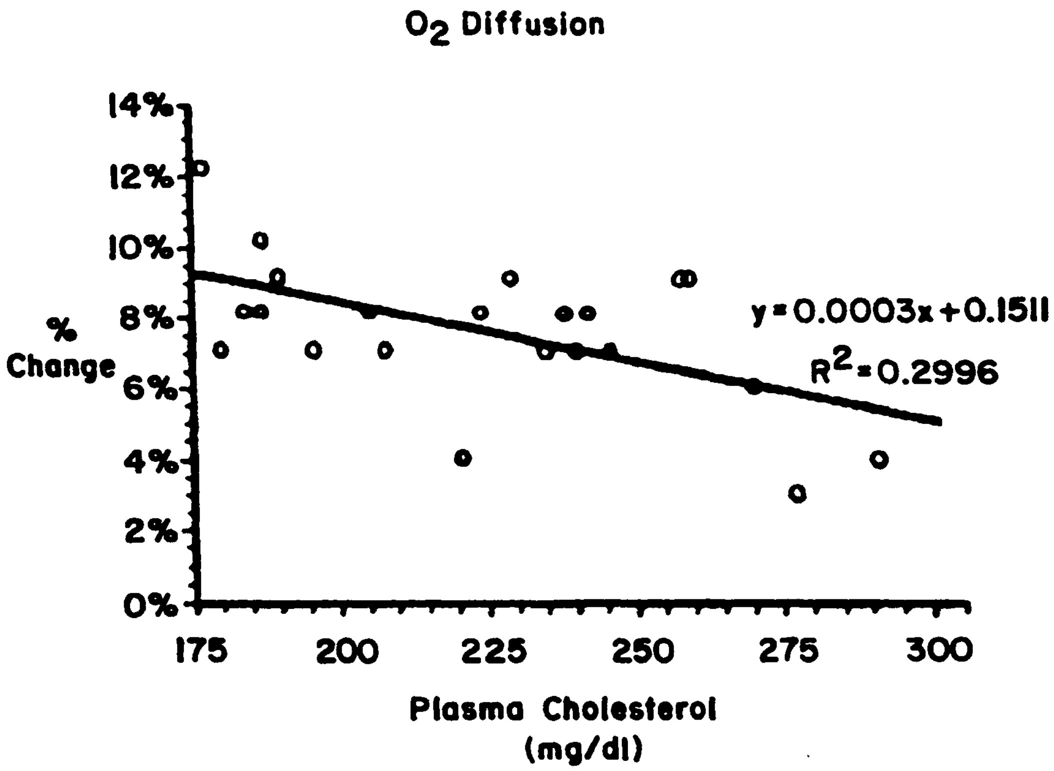

$O_2$ Diffusion
14%
12%
10%
8%
6%
4%
2%
0%
%
Change
y=0.0003x+0.1511
$R^2$=0.2996
175
200
225
250
275
300
Plasma Cholesterol
(mg/dl)


**FIG. 5B**

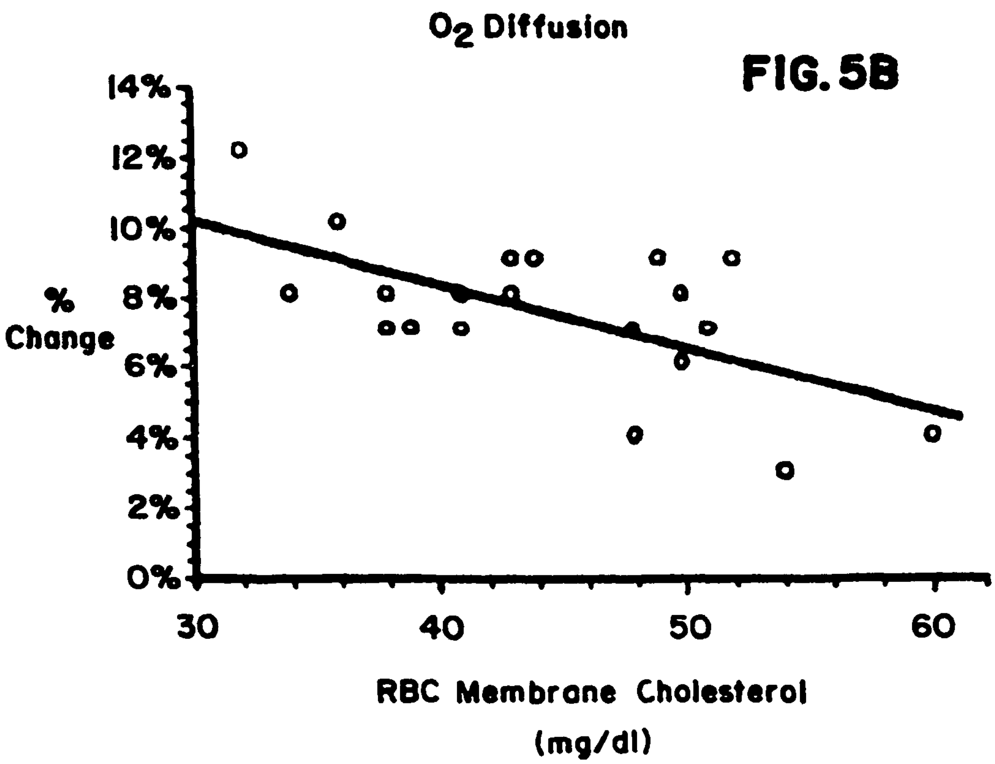

$O_2$ Diffusion
14%
12%
10%
8%
6%
4%
2%
0%
%
Change
30
40
50
60
RBC Membrane Cholesterol
(mg/dl)

FIG. 6

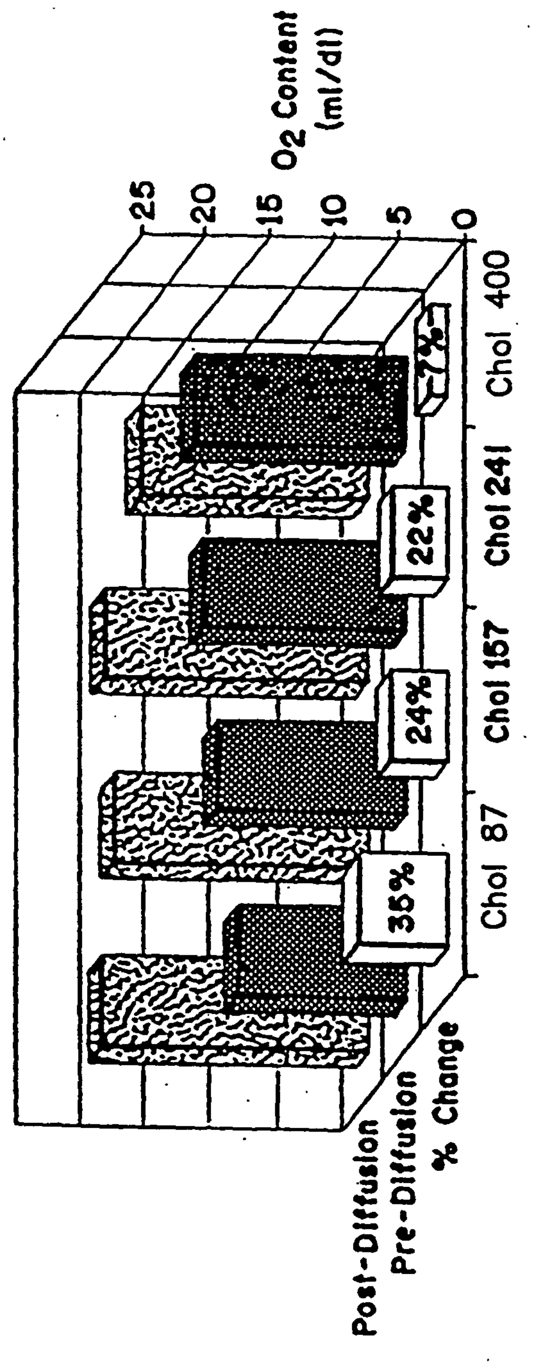
25
20
15
10
5
0
O2 Content
(ml/dl)
35%
24%
22%
7%
Chol 87
Chol 157
Chol 241
Chol 400
Post-Diffusion
Pre-Diffusion
% Change

FIG. 7

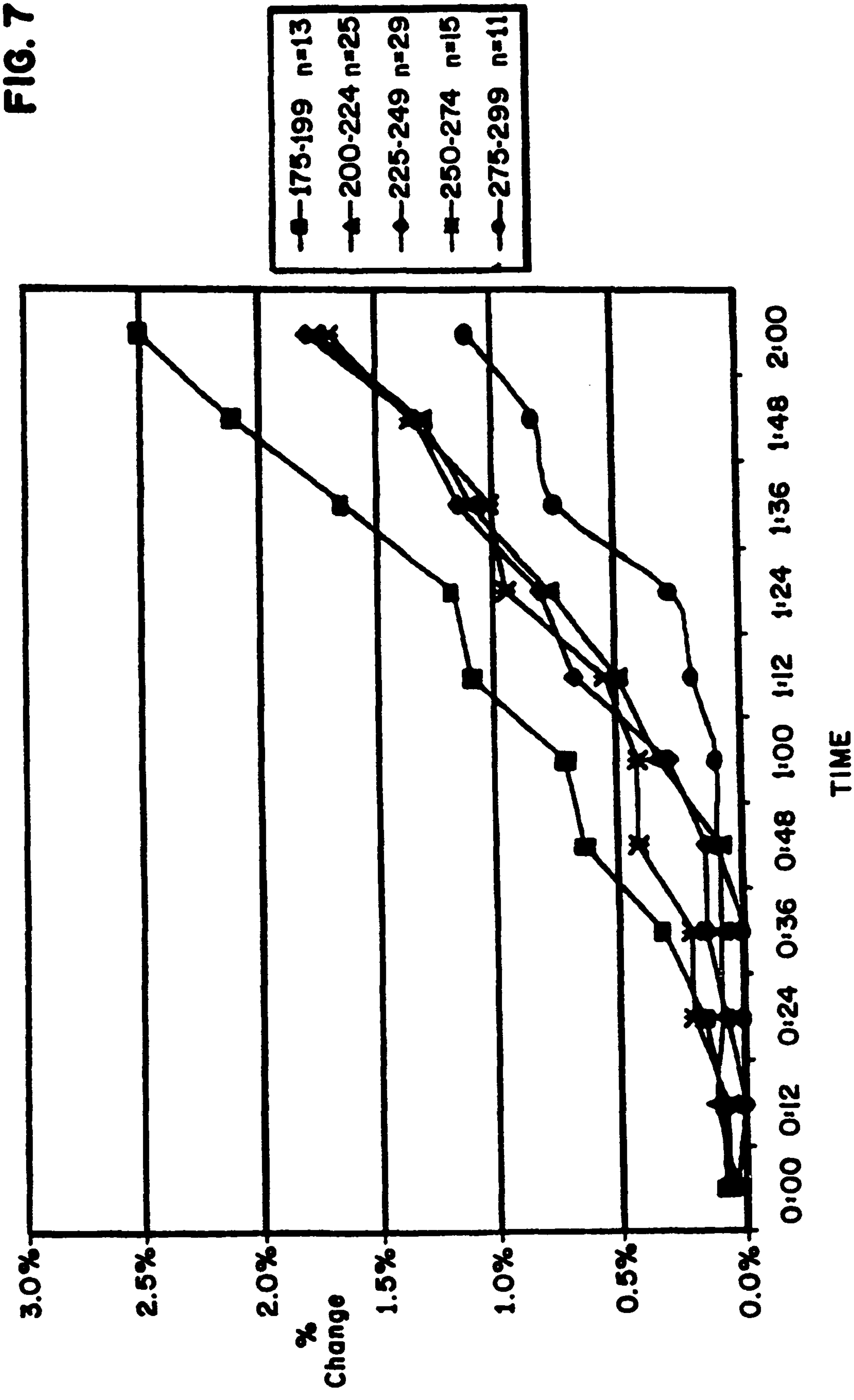
175-199 n=13
200-224 n=25
225-249 n=29
250-274 n=15
275-299 n=11
3.0%
2.5%
2.0%
1.5%
1.0%
0.5%
0.0%
% Change
0:00
0:12
0:24
0:36
0:48
1:00
1:12
1:24
1:36
1:48
2:00
TIME

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- SU 1739295 A
- WO 9921002 A


### Non-patent literature cited in the description


- **STEINBACH J H ; BLACKSHEAR P L JR ; VARCO R L ; BUCHWALD H.** High blood cholesterol reduces in vitro blood oxygen delivery. *THE JOURNAL OF SURGICAL RESEARCH,* February 1974, vol. 16 (2), 134-139
- **KON K ; MAEDA N ; SEKIYA M ; SHIGA T ; SUDA T.** A method for studying oxygen diffusion barrier in erythrocytes: effects of haemoglobin content and membrane cholesterol. *THE JOURNAL OF PHYSIOLOGY,* December 1980, vol. 309, 569-590
- **MENCHACA H J et al.** Decreased blood oxygen diffusion in hypercholesterolemia. *SURGERY,* October 1998, vol. 124 (4), 692-698